(19) Europäisches Patentamt
European Patent Office
Office européen des brevets

(11) EP 3 215 609 B1

(12) # EUROPÄISCHE PATENTSCHRIFT

(45) Veröffentlichungstag und Bekanntmachung des Hinweises auf die Patenterteilung:
**08.04.2020 Patentblatt 2020/15**

(21) Anmeldenummer: **15807781.8**

(22) Anmeldetag: **04.11.2015**

(51) Int Cl.:
***C12N 9/02*** $^{(2006.01)}$ ***C12N 15/82*** $^{(2006.01)}$

(86) Internationale Anmeldenummer:
**PCT/AT2015/000138**

(87) Internationale Veröffentlichungsnummer:
**WO 2016/070206 (12.05.2016 Gazette 2016/19)**

(54) **POLYPEPTID ZUR ENZYMATISCHEN DETOXIFIZIERUNG VON ZEARALENON, SOWIE ISOLIERTES POLYNUKLEOTID, SOWIE ZUSATZSTOFF, VERWENDUNG UND VERFAHREN DESSELBEN**

POLYPEPTIDE FOR THE ENZYMATIC DETOXIFICATION OF ZEARALENONE, ISOLATED POLYNUCLEOTIDE, AND ASSOCIATED ADDITIVE, USE AND METHOD

POLYPEPTIDE DESTINÉ À LA DÉTOXICATION ENZYMATIQUE DU ZÉARALÉNONE, POLYNUCLÉOTIDE ISOLÉ, ET ADDITIF, UTILISATION ET PROCÉDÉ Y RELATIF

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.11.2014 AT 8162014**
**17.08.2015 AT 5382015**

(43) Veröffentlichungstag der Anmeldung:
**13.09.2017 Patentblatt 2017/37**

(73) Patentinhaber: **Erber Aktiengesellschaft**
**3131 Getzersdorf bei Traismauer (AT)**

(72) Erfinder:
- **TORRES ACOSTA, Jean Antonio**
  **1210 Wien (AT)**
- **ADAM, Gerhard**
  **1160 Wien (AT)**
- **KUNZ-VEKIRU, Elisavet**
  **1050 Wien (AT)**
- **MOLL, Wulf-Dieter**
  **2000 Stockerau (AT)**
- **MITTERBAUER, Rudolf**
  **6351 Scheffau am Wilden Kaiser (AT)**
- **SCHMEITZL, Clemens**
  **1180 Wien (AT)**

(74) Vertreter: **Cunow, Gerda**
**Cunow Patentanwalts KG**
**Teschnergasse 33/1/3**
**1180 Wien (AT)**

(56) Entgegenhaltungen:
**EP-A1- 1 455 595 WO-A2-01/42436**

- **MOLNAR O ET AL: "Trichosporon mycotoxinivorans sp. nov., A New Yeast Species Useful in Biological Detoxification of Various Mycotoxins", SYSTEMATIC AND APPLIED MICROBIOLOGY, URBAN & FISCHER, AMSTERDAM, NL, Bd. 27, Nr. 6, 15. Dezember 2004 (2004-12-15), Seiten 661-671, XP004962913, ISSN: 0723-2020, DOI: 10.1078/0723202042369947**
- **SCHATZMAYR G ET AL: "Detoxification of mycotoxins by biotransformation", THE MYCOTOXIN FACTBOOK: FOOD AND FEED TOPICS, X, XX, 1. Januar 2006 (2006-01-01), Seiten 363-375, XP009151517, ISBN: 978-90-8686-006-7**
- **E. VEKIRU ET AL: "Cleavage of Zearalenone by Trichosporon mycotoxinivorans to a Novel Nonestrogenic Metabolite", APPLIED AND ENVIRONMENTAL MICROBIOLOGY, Bd. 76, Nr. 7, 1. April 2010 (2010-04-01), Seiten 2353-2359, XP55276265, US ISSN: 0099-2240, DOI: 10.1128/AEM.01438-09**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- TAKAHASHI-ANDO N ET AL: "A novel lactonohydrolase responsible for the detoxification of zearalenone: enzyme purification and gene cloning", BIOCHEMICAL JOURNAL, PORTLAND PRESS LTD, GB, Bd. 365, 1. Juli 2002 (2002-07-01), Seiten 1-6, XP002967677, ISSN: 0264-6021, DOI: 10.1042/BJ20020450
- ZINEDINE ET AL: "Review on the toxicity, occurrence, metabolism, detoxification, regulations and intake of zearalenone: An oestrogenic mycotoxin", FOOD AND CHEMICAL TOXICOLOGY, PERGAMON, GB, Bd. 45, Nr. 1, 25. November 2006 (2006-11-25), Seiten 1-18, XP005780374, ISSN: 0278-6915, DOI: 10.1016/J.FCT.2006.07.030
- DANIEL E. TORRES PAZMIÑO ET AL: "Self-Sufficient Baeyer-Villiger Monooxygenases: Effective Coenzyme Regeneration for Biooxygenation by Fusion Engineering", ANGEWANDTE CHEMIE INTERNATIONAL EDITION, Bd. 47, Nr. 12, 7. März 2008 (2008-03-07), Seiten 2275-2278, XP55276319, DE ISSN: 1433-7851, DOI: 10.1002/anie.200704630

SEQUENCE LISTING

<110> Erber Aktiengesellschaft

<120> Polypeptid zur Detoxifizierung von Zearalenon, isoliertes Polynukleotid davon sowie einen Zusatzstoff enthaltends Polypeptid, Verwendung desselben

<130> P05435PCT

<140> PCT/AT2015/000138
<141> 2015-11-04

<150> A538/2015
<151> 2015-08-17

<160> 6

<170> PatentIn version 3.5

<210> 1
<211> 630
<212> PRT
<213> Trichosporon mycotoxinivorans

<400> 1

```
Met Thr Leu Glu Ala Ile Pro Val Pro Glu Glu Thr Pro Ala Gln Thr
1               5                   10                  15

Val Pro Thr Ala Pro Leu Ser Glu Glu Glu Arg Glu Ala Leu Lys His
            20                  25                  30

Arg Tyr Arg Ala Glu Arg Asp Lys Arg Ile Arg Glu Asp Gly Ile Lys
        35                  40                  45

Gln Tyr Arg Ser Leu Glu Gly Leu Leu Asp Val Asp Asp Thr Lys Asp
    50                  55                  60

Pro Tyr Thr Pro Val Lys Pro Arg Glu Pro Leu Asn Asp His Val Asp
65                  70                  75                  80

Phe Leu Phe Leu Gly Gly Gly Phe Ala Gly Leu Ile Val Cys Ser His
                85                  90                  95

Leu Lys Lys Thr Gly Phe Asn Asp Phe Arg Ile Ile Glu Ser Gly Gly
            100                 105                 110

Asp Phe Gly Gly Val Trp Tyr Trp Asn Arg Phe Pro Gly Ala Met Cys
        115                 120                 125

Asp Thr Ala Ala Met Val Tyr Leu Pro Leu Leu Glu Glu Thr Gly Thr
    130                 135                 140

Val Pro Ser Ala Lys Tyr Val Arg Gly Pro Glu Ile Leu Ala His Ala
145                 150                 155                 160

Gln Arg Ile Ala Arg Thr Phe Asp Leu Tyr Pro His Ala Leu Phe His
```

```
                165                 170                 175

Thr His Leu Glu Ser Leu Thr Trp Asp Glu Glu Gln Gly Val Trp Lys
            180                 185                 190

Val Lys Thr Arg Gln Gly Asp Ser Phe Thr Ala Thr His Val Gly Met
        195                 200                 205

Gly Thr Gly Pro Leu Asn Lys Pro His Leu Pro Gly Ile Pro Gly Ile
    210                 215                 220

Glu Lys Phe Lys Gly Lys Ala Met His Thr Ala Arg Trp Asp Phe Ala
225                 230                 235                 240

Thr Thr Gly Gly Gly Trp Asn Gly Glu Val Met Glu Gly Leu Lys Asp
                245                 250                 255

Lys Arg Val Gly Val Ile Gly Thr Gly Ala Thr Gly Ile Gln Ala Ile
            260                 265                 270

Pro Glu Leu Gly Arg Asp Ser Gly Glu Leu Phe Val Phe Gln Arg Thr
        275                 280                 285

Pro Ser Ala Val Ala Val Arg Gly Asn His Pro Ile Asp Pro Glu Trp
    290                 295                 300

Phe Ala Ser Leu Asp Lys Gly Trp Gln Thr Lys Trp Asn Arg Asn Phe
305                 310                 315                 320

Ile Gln Leu Met Ser Ser Gly Met Ala Glu Asn Asp Tyr Val Arg Asp
                325                 330                 335

Gly Trp Thr Asp Gly Val Lys Arg Ile Thr Ala Arg Met Phe Ala Glu
            340                 345                 350

Ala Ala Lys Ala Gly Arg Asp Pro Ser Thr Leu Ser Phe Asp Asp Phe
        355                 360                 365

Leu Ala Ala Tyr His Leu Ser Asp Asp Glu Tyr Met Thr Ala Val Arg
    370                 375                 380

Asn Arg Thr Asn Glu Val Val Asn Asp Pro Lys Thr Ala Asp Gly Leu
385                 390                 395                 400

Lys Ala Trp Tyr Arg Gln Phe Cys Lys Arg Pro Cys Phe His Asp Glu
                405                 410                 415

Phe Leu Ala Thr Phe Asn Arg Pro Thr Val His Leu Val Asp Thr Asn
            420                 425                 430

Gly Gln Gly Val Thr Glu Val Asp Glu Thr Gly Val Trp Ala Asn Gly
```

435 440 445

Gln His Tyr Asp Leu Asp Ile Ile Val Tyr Ala Thr Gly Phe Glu Phe
450 455 460

Asn Ser Glu Trp Thr Tyr Lys Ser Gly Met Glu Val Tyr Gly Arg Asp
465 470 475 480

Gly Leu Thr Leu Thr Gln Ala Trp Lys Asp Gly Met Lys Thr Tyr His
485 490 495

Gly Met His Ile Asn Gly Phe Pro Asn Leu Phe Met Leu Gln Phe Gln
500 505 510

Gln Gly Ser Ser Leu Ala Ser Asn Ile Thr Ser Asn Tyr Val Asp Ser
515 520 525

Gly Tyr Thr Ile Ala Ala Ile Leu Asn Lys Lys Lys Glu Leu Gly Ala
530 535 540

Lys Thr Val Glu Val Pro Ala Asp Val Gln Ser Lys Trp Ile Glu His
545 550 555 560

Leu Leu Thr Ala Asn Lys Gly Leu Ile Gly Gly Pro Glu Cys Thr Pro
565 570 575

Gly Tyr Tyr Asn Asn Glu Gly Gln Glu Glu Gly Leu Lys Glu Lys Leu
580 585 590

Asn Gly Ala Arg Tyr Pro Ala Gly Ser Leu Ala Phe Phe Asp Tyr Ile
595 600 605

Ala Glu Trp Arg Thr Asn Gly Lys Phe Glu Gly Leu Ala Phe Asp Gly
610 615 620

Lys Gln Ile Ala Val Gln
625 630

<210> 2
<211> 709
<212> PRT
<213> Trichosporon mycotoxinivorans

<400> 2

```
Met Ser Ala Ser Arg Pro Arg Gly Ser Pro His Tyr Leu Gly Asp Arg
1               5                   10                  15

Ser Thr Lys Ser Gly Leu Arg Arg Pro Phe Lys Arg Ser Lys Lys Asn
            20                  25                  30

Arg Ala Thr Asn Ala Val Phe Ser Gln Arg His Arg Lys Pro Thr Glu
        35                  40                  45
```

```
Gly Ala Gly Val Arg Gly Lys His Ser Cys Ser Val Ile Pro Asp Lys
    50                  55                  60

His Thr Ala Pro Trp Gly Pro Ala Ala Ala Glu Pro Gly Trp His Met
65                  70                  75                  80

Thr Leu Glu Ala Ile Pro Val Pro Glu Glu Thr Pro Ala Gln Thr Val
                85                  90                  95

Pro Thr Ala Pro Leu Ser Glu Glu Glu Arg Glu Ala Leu Lys His Arg
            100                 105                 110

Tyr Arg Ala Glu Arg Asp Lys Arg Ile Arg Glu Asp Gly Ile Lys Gln
        115                 120                 125

Tyr Arg Ser Leu Glu Gly Leu Leu Asp Val Asp Asp Thr Lys Asp Pro
    130                 135                 140

Tyr Thr Pro Val Lys Pro Arg Glu Pro Leu Asn Asp His Val Asp Phe
145                 150                 155                 160

Leu Phe Leu Gly Gly Gly Phe Ala Gly Leu Ile Val Cys Ser His Leu
                165                 170                 175

Lys Lys Thr Gly Phe Asn Asp Phe Arg Ile Ile Glu Ser Gly Gly Asp
            180                 185                 190

Phe Gly Gly Val Trp Tyr Trp Asn Arg Phe Pro Gly Ala Met Cys Asp
        195                 200                 205

Thr Ala Ala Met Val Tyr Leu Pro Leu Leu Glu Glu Thr Gly Thr Val
    210                 215                 220

Pro Ser Ala Lys Tyr Val Arg Gly Pro Glu Ile Leu Ala His Ala Gln
225                 230                 235                 240

Arg Ile Ala Arg Thr Phe Asp Leu Tyr Pro His Ala Leu Phe His Thr
                245                 250                 255

His Leu Glu Ser Leu Thr Trp Asp Glu Glu Gln Gly Val Trp Lys Val
            260                 265                 270

Lys Thr Arg Gln Gly Asp Ser Phe Thr Ala Thr His Val Gly Met Gly
        275                 280                 285

Thr Gly Pro Leu Asn Lys Pro His Leu Pro Gly Ile Pro Gly Ile Glu
    290                 295                 300

Lys Phe Lys Gly Lys Ala Met His Thr Ala Arg Trp Asp Phe Ala Thr
305                 310                 315                 320
```

```
Thr Gly Gly Gly Trp Asn Gly Glu Val Met Glu Gly Leu Lys Asp Lys
                325                 330                 335

Arg Val Gly Val Ile Gly Thr Gly Ala Thr Gly Ile Gln Ala Ile Pro
            340                 345                 350

Glu Leu Gly Arg Asp Ser Gly Glu Leu Phe Val Phe Gln Arg Thr Pro
        355                 360                 365

Ser Ala Val Ala Val Arg Gly Asn His Pro Ile Asp Pro Glu Trp Phe
    370                 375                 380

Ala Ser Leu Asp Lys Gly Trp Gln Thr Lys Trp Asn Arg Asn Phe Ile
385                 390                 395                 400

Gln Leu Met Ser Ser Gly Met Ala Glu Asn Asp Tyr Val Arg Asp Gly
                405                 410                 415

Trp Thr Asp Gly Val Lys Arg Ile Thr Ala Arg Met Phe Ala Glu Ala
            420                 425                 430

Ala Lys Ala Gly Arg Asp Pro Ser Thr Leu Ser Phe Asp Asp Phe Leu
        435                 440                 445

Ala Ala Tyr His Leu Ser Asp Asp Glu Tyr Met Thr Ala Val Arg Asn
    450                 455                 460

Arg Thr Asn Glu Val Val Asn Asp Pro Lys Thr Ala Asp Gly Leu Lys
465                 470                 475                 480

Ala Trp Tyr Arg Gln Phe Cys Lys Arg Pro Cys Phe His Asp Glu Phe
                485                 490                 495

Leu Ala Thr Phe Asn Arg Pro Thr Val His Leu Val Asp Thr Asn Gly
            500                 505                 510

Gln Gly Val Thr Glu Val Asp Glu Thr Gly Val Trp Ala Asn Gly Gln
        515                 520                 525

His Tyr Asp Leu Asp Ile Ile Val Tyr Ala Thr Gly Phe Glu Phe Asn
    530                 535                 540

Ser Glu Trp Thr Tyr Lys Ser Gly Met Glu Val Tyr Gly Arg Asp Gly
545                 550                 555                 560

Leu Thr Leu Thr Gln Ala Trp Lys Asp Gly Met Lys Thr Tyr His Gly
                565                 570                 575

Met His Ile Asn Gly Phe Pro Asn Leu Phe Met Leu Gln Phe Gln Gln
            580                 585                 590
```

```
Gly Ser Ser Leu Ala Ser Asn Ile Thr Ser Asn Tyr Val Asp Ser Gly
        595                 600                 605

Tyr Thr Ile Ala Ala Ile Leu Asn Lys Lys Lys Glu Leu Gly Ala Lys
    610                 615                 620

Thr Val Glu Val Pro Ala Asp Val Gln Ser Lys Trp Ile Glu His Leu
625                 630                 635                 640

Leu Thr Ala Asn Lys Gly Leu Ile Gly Gly Pro Glu Cys Thr Pro Gly
                645                 650                 655

Tyr Tyr Asn Asn Glu Gly Gln Glu Glu Gly Leu Lys Glu Lys Leu Asn
            660                 665                 670

Gly Ala Arg Tyr Pro Ala Gly Ser Leu Ala Phe Phe Asp Tyr Ile Ala
        675                 680                 685

Glu Trp Arg Thr Asn Gly Lys Phe Glu Gly Leu Ala Phe Asp Gly Lys
    690                 695                 700

Gln Ile Ala Val Gln
705
```

<210> 3
<211> 630
<212> PRT
<213> Trichosporon asahii

<400> 3

```
Met Thr Ile Asp His Ile Pro Glu Pro Thr Asp Tyr Lys Gln Thr Gln
1               5                   10                  15

Pro Val Ala Pro Leu Ala Asp Glu Glu Arg Gln Ala Leu Gln Ala Lys
            20                  25                  30

Tyr Arg Glu Glu Arg Asp Lys Arg Leu Arg Ala Asp Gly Ile Asn Gln
        35                  40                  45

Tyr Lys Pro Leu Gly Gly Ile Leu Lys Leu Asp Glu Asp Lys Asp Pro
    50                  55                  60

Tyr Thr Asp Val Gln Pro Arg Glu Pro Val His Asp His Val Asp Phe
65                  70                  75                  80

Leu Phe Leu Gly Gly Gly Phe Ala Gly Leu Thr Val Cys Ala Lys Leu
                85                  90                  95

Lys Gln Ala Gly Phe Asp Ser Ile Arg Ile Leu Glu Ser Gly Gly Asp
            100                 105                 110
```

```
Phe Gly Gly Val Trp Tyr Trp Asn Arg Phe Pro Gly Ala Met Cys Asp
        115                 120                 125

Thr Ala Ala Met Val Tyr Leu Pro Met Met Glu Glu Val Gly Thr Val
    130                 135                 140

Pro Ser Ala Lys Tyr Val Arg Gly Pro Glu Ile Leu Ala His Ala His
145                 150                 155                 160

Arg Ile Ala Arg His Phe Asp Leu Tyr Pro His Ala Leu Phe Ser Thr
                165                 170                 175

His Leu Glu Glu Leu Arg Trp Asp Glu Asp Arg Ser Val Tyr Val Val
            180                 185                 190

Lys Thr Arg Glu Gly Asp Glu Phe Thr Ala Thr Asn Val Ala Met Gly
        195                 200                 205

Thr Gly Pro Leu Asn Arg Pro His Leu Pro Gly Ile Pro Gly Leu Glu
    210                 215                 220

Thr Phe Lys Gly Gln Ala Met His Thr Ala Arg Trp Asp Phe Gly Val
225                 230                 235                 240

Thr Gly Gly Gly Trp Asp Asp Glu Val Leu Glu Gly Leu Lys Asp Lys
                245                 250                 255

Arg Val Gly Val Ile Gly Thr Gly Ala Thr Gly Val Gln Cys Ile Pro
            260                 265                 270

Thr Leu Gly Arg Asp Ser Gly Ser Leu His Val Phe Gln Arg Thr Pro
        275                 280                 285

Ser Ala Val Ala Ile Arg Gly Asn His Ala Ile Asp Lys Glu Trp Phe
    290                 295                 300

Ser Gln Leu Asp Lys Gly Trp Gln Thr Lys Trp Leu Arg Asn Phe Cys
305                 310                 315                 320

Gln Leu Met Ser Thr Gly Tyr Ala Pro Val Asp Tyr Val His Asp Gly
                325                 330                 335

Trp Thr Asp Gly Val Gln Arg Ile Thr Lys Arg Met Leu Glu Met Cys
            340                 345                 350

Ala Lys Glu Gly Arg Ala Pro Thr Gly Met Ala Asp Tyr Met Lys Ala
        355                 360                 365

Tyr His Leu Ser Asp Asp Glu Tyr Thr Thr Ala Val Arg Ala Arg Thr
    370                 375                 380
```

```
Asp Glu Val Val Lys Asp Pro Glu Thr Ala Asp Lys Leu Lys Ala Trp
385                 390                 395                 400

Tyr Arg Gln Phe Cys Lys Arg Pro Thr Phe His Asp Glu Tyr Leu Gln
                405                 410                 415

Thr Phe Asn Arg Pro Asn Val Thr Leu Val Asp Thr Asp Gly Lys Gly
            420                 425                 430

Val Glu Arg Ile Asp Glu Thr Gly Val Trp Ala Asn Gly Lys His Tyr
        435                 440                 445

Asp Leu Asp Val Leu Val Tyr Ala Thr Gly Phe Glu Phe Asn Ser Glu
    450                 455                 460

Tyr Thr Tyr Lys Ser Gly Leu Glu Val Tyr Gly Arg Gly Gly Arg Thr
465                 470                 475                 480

Leu Thr Asp Ala Trp Lys Asp Gly Met Gln Ser Phe Gln Gly Met His
                485                 490                 495

Val His Gly Phe Pro Asn Leu Phe Val Ile Gly Phe Ala Gln Gly Ser
            500                 505                 510

Ser Leu Ala Ser Asn Ile Thr Ser Asn Tyr Thr Glu His Gly Pro Thr
        515                 520                 525

Val Leu Ser Ile Leu Gln Lys Lys Lys Glu Leu Gly Ala Lys Thr Val
    530                 535                 540

Glu Val Pro Gln Lys Thr Gln Asp Asp Trp Ile Glu Leu Ile Leu Gln
545                 550                 555                 560

Gly Asp Arg Gly Ile Ile Gly Gly Pro Glu Cys Thr Pro Gly Tyr Tyr
                565                 570                 575

Asn Asn Glu Gly Gln Glu Glu Gly Arg Arg Glu Arg Leu Asn Val Ala
            580                 585                 590

Arg Tyr Pro Ala Gly Pro Leu Ala Phe Phe Asp Tyr Ile Ala Glu Trp
        595                 600                 605

Arg Ala Asn Gly Lys Phe Glu Gly Leu Glu Phe Asp Gly Lys Pro Val
    610                 615                 620

Glu Ile Ala Ala Thr Leu
625                 630
```

<210> 4
<211> 1890
<212> DNA
<213> Artificial Sequence

<220>
<223> Codon Optimiertes Polynukleotid des Polypeptids mit SEQ ID Nr. 1 zur Expression in Saccharomyces cerevisiae

<400> 4

```
atgactttgg aagctattcc agttccagaa gaaactccag ctcaaactgt tccaactgct      60
ccattgtctg aagaagaaag agaagccttg aaacatagat acagagccga aagagataag     120
agaatcagag aagatggtat caagcaatac agatccttgg aaggtttgtt ggatgttgat     180
gataccaaag atccatacac tccagttaag ccaagagaac cattgaacga tcatgtcgat     240
tttttgtttt tgggtggtgg ttttgctggt ttgatcgttt gttctcattt gaaaaagacc     300
ggtttcaacg acttcagaat cattgaatct ggtggtgatt tcggtggtgt ttggtattgg     360
aatagatttc caggtgctat gtgtgatact gctgctatgg tttatttgcc tttgttggaa     420
gaaactggta cagttccatc tgctaaatat gttagaggtc cagaaatttt ggctcacgct     480
caaagaattg ctagaacttt tgacttgtac ccacatgctt tgttccatac ccatttggaa     540
tctttgactt gggatgaaga acaaggtgtt tggaaagtta agaccagaca aggtgattct     600
ttcactgcta ctcatgttgg tatgggtact ggtccattga acaaaccaca tttgcctggt     660
attccaggta tcgaaaagtt taaaggtaag gctatgcata ccgctagatg ggattttgct     720
actactggtg gtggttggaa tggtgaagtt atggaaggtt taaaggataa gagagttggt     780
gttattggta ctggtgctac tggtattcaa gctataccag aattgggtag agactccggt     840
gaattatttg ttttccaaag aacaccatcc gctgttgcag ttagaggtaa tcatccaatt     900
gatccagaat ggtttgcctc tttggataag ggttggcaaa caaagtggaa cagaaacttt     960
attcaattga tgtcctccgg tatggccgaa aatgattacg ttagagatgg ttggactgat    1020
ggtgttaaga gaattactgc tagaatgttt gctgaagctg ctaaagctgg tagagatcca    1080
tctactttgt ctttcgatga tttcttggct gcctaccatt tgtccgatga tgaatatatg    1140
actgccgtca gaaacagaac taacgaagtt gttaacgatc caaagactgc tgatggtttg    1200
aaagcttggt atagacaatt ctgcaaaaga ccatgcttcc acgatgaatt tttggctact    1260
tttaacagac caaccgttca cttggttgat acaaatggtc aaggtgttac tgaagttgac    1320
gaaaccggtg tttgggctaa tggtcaacat tacgatttgg atattatcgt ttacgccact    1380
ggtttcgaat tcaactctga atggacttac aagtctggta tggaagttta tggtagagat    1440
ggtttgacat tgactcaagc ttggaaagac ggtatgaaaa cctatcatgg tatgcacatt    1500
aacggtttcc caaacttgtt catgttgcaa ttccaacaag gttcctcttt ggcttctaac    1560
atcacttcta actacgttga ttccggttac accattgctg ctattttgaa caaaaagaaa    1620
gaattaggtg ccaagaccgt tgaagttcca gctgatgttc aatctaaatg gatcgaacat    1680
ttgttgaccg ctaacaaggg tttgattggt ggtccagaat gtactccagg ttactataac    1740
aatgaaggtc aagaagaagg tttgaaagaa aagttgaacg gtgctagata tccagctggt    1800
tcattggctt ttttcgatta cattgctgaa tggagaacta atggtaaatt cgaaggtttg    1860
```

gccttcgatg gtaaacaaat tgctgttcaa 1890

<210> 5
<211> 2127
<212> DNA
<213> Trichosporon mycotoxinivorans

<400> 5

```
atgtctgcta gtagaccgcg cggctcccca cattatctcg gtgaccggtc taccaaaagc     60
ggtctacgca gaccgtttaa acggtcgaaa aagaacagag ccacgaacgc cgtgttttcc    120
cagcgccatc gcaagccaac cgaaggtgcc ggtgttcggg gtaaacacag ttgctcggtg    180
atccccgata agcacaccgc cccatggggc ccggccgccg ccgaacctgg atggcacatg    240
acactcgaag ctattcctgt tcccgaggag acgcccgcgc agaccgtccc caccgctccg    300
ctctcggagg aagagcgcga ggccttgaag caccgatacc gcgccgagag ggacaagcgt    360
atccgcgaag acggcatcaa gcagtaccgc tccctggagg gtcttctgga cgtcgacgac    420
accaaggacc cgtacacgcc cgtgaagcct cgtgagcccc tgaacgacca tgtcgacttc    480
ctgttcctgg gtggtgggtt cgccggcctc attgtctgct cccatctcaa aaagaccggc    540
ttcaacgatt tccgcatcat cgagtcgggc ggagactttg gaggtgtatg gtactggaac    600
cgcttccccg gcgccatgtg tgacaccgcc gcgatggtct acttgcccct cctcgaggag    660
acgggcaccg tgccgtcggc caagtatgtc cgtggcccag agattcttgc gcacgcgcag    720
cgcatcgctc gcacgtttga cctgtacccg cacgctctgt tccacacgca cctcgagtct    780
cttacgtggg acgaggagca gggcgtctgg aaggtcaaga cgcgccaggg cgactcgttc    840
acggcgaccc acgtgggcat gggcaccgga cctctcaaca agccccacct tcctggtatc    900
cccggtatcg aaaagttcaa gggcaaggcg atgcacactg cccgctggga ctttgcgacg    960
acgggcggcg gctggaatgg agaagttatg gagggactca aggacaagcg cgtcggcgtg   1020
atcggtaccg gcgctaccgg catccaggcg attcccgagc tcggccgcga cagcggcgag   1080
cttttcgtgt tccagcgcac gccctccgct gtcgccgtcc gaggcaacca ccccatcgac   1140
cccgagtggt ttgcgagcct cgacaagggg tggcagacaa agtggaaccg taactttatc   1200
cagctcatga gctcgggcat ggccgagaac gactatgtcc gtgacggctg gaccgacggc   1260
gtcaagcgca ttacggccag gatgtttgcc gaggcggcaa aggctggtcg ggacccttcg   1320
actctgtcgt ttgatgactt ccttgcggcg taccatctct cggacgacga gtacatgaca   1380
gcggtacgca accgcactaa cgaagtcgtc aacgacccca agaccgccga cggtctcaag   1440
gcgtggtacc gccagttttg caagcgtccg tgcttccacg acgagttttt ggccaccttc   1500
aaccgcccta ccgtccacct cgtggacacc aacggccagg gcgtaaccga ggtcgacgag   1560
acgggcgtct gggccaacgg acagcactac gacctcgaca ttatcgtcta tgccactgga   1620
ttcgagttca actcggagtg gacatacaag tccggcatgg aggtgtacgg ccgcgacggg   1680
ctcaccctca cgcaggcgtg gaaggacggc atgaagacgt accacggcat gcacatcaac   1740
```

gggttcccca acctgttcat gcttcagttc cagcagggat cgtcgctcgc atccaacatt 1800
acatccaact atgtcgactc gggctacacc attgcggcga ttctcaacaa gaaaaaggag 1860
ctcggagcca agacggtcga ggtccccgcc gacgtccagt caaagtggat cgagcacctc 1920
ctcaccgcga acaagggcct gatcggagga ccagagtgca cccccggata ctacaacaac 1980
gagggccagg aggaaggcct caaggagaag ctcaacggag cacggtaccc tgcgggatct 2040
ctcgcattct ttgactacat cgccgagtgg cgtacaaacg gcaagtttga gggtcttgcg 2100
tttgatggaa agcagatcgc agtgcag 2127

<210> 6
<211> 1893
<212> DNA
<213> Trichosporon asahii

<400> 6

```
atgacgatcg accacatccc agagccgacg gactacaagc agacgcagcc cgtcgcgcct      60
ctggcggacg aggagcgcca ggcgctgcag gccaaatacc gcgaggagcg cgacaagcga     120
ctccgcgccg atggtattaa ccagtacaag cccctcggcg gcatcttgaa gctggacgag     180
gacaaggacc cgtacacgga cgtccagccg cgcgagccgg tccacgacca tgtcgacttt     240
ctgttccttg gcggtggctt cgcgggcctg acggtgtgcg cgaagctgaa gcaggcgggc     300
tttgactcga tccgcatcct cgagagcgga ggcgactttg gcggcgtctg gtactggaac     360
cgcttccccg gcgccatgtg cgacacggcc gccatggtct acctccccat gatggaggag     420
gttggcacgg tgccctcggc caaatatgtc cgcggacccg agattctcgc ccacgcccac     480
cgcatcgcgc gccacttcga cctctacccc cacgctctgt tcagcaccca cctcgaggag     540
ctgcgctggg acgaggaccg cagcgtctac gttgtcaaga cgcgcgaggg cgacgagttc     600
acggcgacca acgtcgcgat gggcactgga cccctgaacc gtccccacct gcccggtatc     660
cctggactgg agacattcaa gggccaggcg atgcacaccg ctcgctggga ctttggcgtg     720
accggcggag gctgggacga tgaggtcctc gaggggttga aggacaagcg tgtcggtgtc     780
atcggcacgg gcgccacggg tgtgcagtgt atcccgactc ttggacgcga ctctggctcc     840
ctgcatgtgt tccagcgcac gccctctgcc gtcgctatcc gcggcaacca tgcgattgac     900
aaggagtggt tcagccagct ggacaagggg tggcagacca agtggctgcg caacttctgc     960
cagctcatgt cgaccgggta cgcccccgtc gattacgtcc acgacgggtg gaccgacggc    1020
gtgcagcgca tcacgaagcg catgctcgag atgtgcgcca aggaggggcg cgcgccgacc    1080
ggaatggcag actacatgaa ggcgtaccac ctcagcgacg acgagtacac caccgccgtg    1140
cgcgcccgca ccgacgaggt cgtcaaggat cccgaaacgg ccgacaagct gaaggcgtgg    1200
tacaggcagt tttgcaaacg ccccaccttt catgacgagt acttgcagac gttcaaccgg    1260
ccgaacgtta cgctcgtcga cacggacggc aagggcgtgg agcgcatcga cgagacgggc    1320
gtctgggcga acggcaagca ctacgacctc gacgtacttg tgtacgcgac aggcttcgag    1380
ttcaactcgg agtacacata caagtctggt ctcgaggtgt acggtcgcgg tgggcgcacg    1440

ctcaccgatg catggaagga cggcatgcag tccttccagg gcatgcacgt gcacgggttc    1500
ccgaacctgt tcgtgatcgg ttttgcgcag gggtcctcac tcgcgtccaa cattacgagc    1560
aactacaccg agcacgggcc cacggtcctc agcatcctgc agaagaagaa ggagctcggc    1620
gctaagacgg tcgaggtgcc ccagaaaacg caggacgact ggatcgaact catcctccag    1680
ggcgaccggg gcattattgg cggtcccgag tgcacacccg gctactacaa caacgagggc    1740
caggaggagg gccgtcgcga gcgcctcaat gttgcgcgat accccgctgg tccgctcgct    1800
ttcttcgact acatcgccga gtggcgcgcg aacggcaagt tcgagggcct cgagtttgat    1860
ggcaagccgg tcgagattgc cgcgactctg tag                                 1893
```

## Beschreibung

Die vorliegende Erfindung bezieht sich auf ein Polypeptid zur enzymatischen Detoxifizierung von Zearalenon, eine transgene Wirtszelle zur Herstellung einer Zearalenon detoxifizierenden Monooxygenase sowie auf eine Verwendung eines isolierten Polynukleotids sowie auf ein Verfahren zur Herstellung einer Zearalenon detoxifizierenden Monooxygenase.

Mykotoxine sind von filamentösen Pilzen produzierte Sekundärmetabolite. Ein wichtiger Vertreter derselben ist das weltweit verbreitete Zearalenon (ZEN), früher bekannt als F-2 Toxin, das durch eine Vielzahl von Fusarien-Pilzen produziert wird. Diese Pilze befallen unter anderem Kulturpflanzen, wie verschiedene Getreidearten, wobei in der Regel der Pilzbefall vor der Ernte auftritt, wobei das Pilzwachstum bzw. die Mykotoxinproduktion vor oder bei nicht sachgemäßer Lagerung auch nach der Ernte erfolgen kann. Die FAO schätzt, dass weltweit 25 % der agrarischen Produkte mit Mykotoxinen kontaminiert sind, was zu erheblichen wirtschaftlichen Einbußen führt. In einer kürzlich weltweit durchgeführten Studie wurden von Jänner 2009 bis 2011 insgesamt 23.781 Proben analysiert, wobei 81 % positiv auf mindestens ein Mykotoxin und 45 % positiv auf ZEN getestet wurden. ZEN konnte in allen Regionen der Welt ebenso wie in allen getesteten Getreide- und Futtermittelklassen, wie beispielsweise Mais, Sojamehl, Weizen, Weizenkleie, DDGS (Trockenschlempe) sowie in Fertigfuttermischungen mit einer Häufigkeit von bis zu 100 % gefunden werden.

ZEN ist ein nicht steroides, östrogenes, makrozyklisches, über den Polyketid-Stoffwechselweg synthetisiertes Lacton mit der Strukturformel:

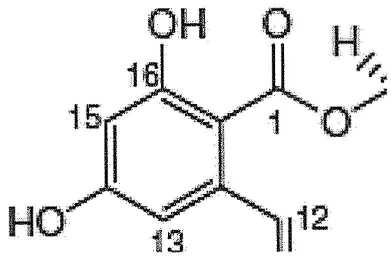

und dem IUPAC Nomenklaturnamen (2E,11S)-15,17-dihydroxy-11-methyl-12-oxabicyclo[12.4.0] octadeca-1(18),2,14,16-tetraene-7,13-dion. Für ZEN und ZEN-Metabolite gibt es unterschiedliche Nomenklaturen, wobei in diesem Dokument soweit wie möglich die Nomenklatur von Metzler (2011, Mycotox. Res., 27:1-3) übernommen wird. In der Natur kommen neben ZEN auch eine Vielzahl von ZEN-Derivaten vor, die durch enzymatische oder chemische Modifikationen von ZEN entstehen. Weiterhin werden ZEN-Metabolite unter anderem im menschlichen oder tierischen Organismus gebildet.

ZEN ebenso wie auch ZEN-Derivate, wie α-ZEL oder β-ZEL können aufgrund ihrer hohen chemischen und physikalischen Stabilität auch in verarbeiteten Nahrungs- oder Futtermitteln, wie z.B. Brot, Bier oder Trockenschlempe nachgewiesen werden.

Obwohl ZEN eine relativ geringe akute Toxizität aufweist und einen oralen LD50-Wert von bis zu 20.000 mg/kg Körpergewicht besitzt, können bei länger dauernder Aufnahme subakute und/oder subchronische toxische Wirkungen, wie beispielsweise teratogene, karzinogene, immunsupressive und östrogene Wirkungen bei Tieren oder Menschen auftreten. ZEN bindet an den Östrogenrezeptor und kann hormonelle Störungen verursachen, daher wird im Allgemeinen mit Detoxifizierung bzw. Detoxifikation von Zearalenon eine Herabsetzung der des gebildeten Metaboliten im Vergleich zu ZEN verstanden.

Die Aufnahme von mit ZEN kontaminiertem Futtermittel führt zu Entwicklungsstörungen bei Säugetieren, wobei Schweine, und im speziellen Jungtiere äußerst sensitiv gegenüber ZEN sind. ZEN-Konzentrationen im Futtermittel von über 0,5 ppm führen zu Entwicklungsstörungen, wobei z.B. Konzentrationen von über 1,5 ppm zu Hyperöstrogenität bei Schweinen führen können und Konzentrationen von 12 ppm ZEN für Fehlgeburten bei Rindern verantwortlich gemacht wurden. Da Zearalenon schnell über die Schleimhäute, insbesondere über die Magen- aber auch über die Mundschleimhaut aufgenommen wird, ist eine unverzügliche und vor allem quantitative Detoxifizierung notwendig. Bereits 30 Minuten nach oraler Gabe von ZEN kann dieses im Blut nachgewiesen werden. Um eine möglichst vollständige Detoxifizierung zu erreichen hat sich der Einsatz isolierter Enzyme gegenüber Mikroorganismen als vorteilhaft erwiesen, da sie eine höhere spezifische Aktivität oder eine schnellere Wirkung aufweisen. Aufgrund der schädlichen Wirkung von ZEN gibt es in der Europäischen Union verbindliche ZEN-Obergrenzen in Nahrungsmitteln sowie Empfehlungen für ZEN-Obergrenzen in Futtermitteln (EC NO: 1881/2006).

Die primäre Strategie, um die ZEN-Kontamination von Nahrungsmitteln oder Futtermitteln zu reduzieren, ist die Einschränkung des Pilzwachstums, beispielsweise durch Einhaltung der "guten landwirtschaftlichen Praxis". Dazu zählt unter anderem, dass Saatgut frei von Schädlingen und Pilzbefall ist, oder dass landwirtschaftliche Abfallprodukte

rechtzeitig vom Feld entfernt werden. Des Weiteren kann durch den Einsatz von Fungiziden das Pilzwachstum auf dem Feld reduziert werden. Nach der Ernte sollte das Erntegut bei einer Restfeuchtigkeit von unter 15 % und geringer Temperatur gelagert werden, um das Pilzwachstum zu verhindern. Ebenso sollte vor der Weiterverarbeitung durch Pilzbefall kontaminiertes Gut entfernt werden. Trotz dieser Liste von Maßnahmen berichteten I. Rodriges und K. Naehrer (2012), dass selbst in Regionen mit den höchsten landwirtschaftlichen Standards, wie den USA und Zentraleuropa in den Jahren 2009 bis 2011 29 % bzw. 39 % der getesteten Maisproben mit ZEN kontaminiert waren.

Weitere Möglichkeiten zur Entfernung von ZEN aus Futter- oder Nahrungsmitteln sind die Adsorption bzw. die Transformation des Mykotoxins. Hierfür ist es erforderlich, dass die Bindung des Mykotoxins zum Adsorbens über einen weiten pH-Bereich stark und spezifisch ist und während des gesamten Verdauungsprozesses im gastrointestinalen Bereich stabil bleibt. Obwohl einige nicht-biologische Adsorptionsmittel, wie z.B. Aktivkohle, Silikate oder synthetische Polymere, wie Cholestryamin, für Aflatoxine effizient eingesetzt werden können, ist ihr Einsatz für andere Mykotoxine beschränkt. Der wesentliche Nachteil der Adsorptionsmittel ist die nicht-spezifische Bindung von anderen Molekülen, die teilweise essentiell für die Ernährung sind. Biologische Adsorptionsmittel, wie z.B. Hefe oder Hefeextrakte sind in der Literatur ebenfalls beschrieben, sind jedoch ähnlich limitiert wie nicht-biologische Adsorptionsmittel.

Auch die Detoxifizierung von ZEN durch physikalische und chemische Behandlungen ist limitiert. Durch thermische Behandlung kann ZEN nicht effektiv deaktiviert werden, allerdings kann der ZEN-Gehalt durch Extrusion und Behandlung mit Oxidationsmitteln, z.B. für 16 h bei 80 °C mit 10 %iger Wasserstoffperoxidlösung um 83,9 % reduziert werden. Der Einsatz von Extrusionsverfahren und Oxidationsmitteln, wie Ozon oder Wasserstoffperoxid in der Futter- und Nahrungsmittelherstellung ist auf Grund der hohen Kosten, dem Qualitätsverlust, der teilweise geringen Effizienz und der geringen Spezifität begrenzt.

Aus der EP 0 938 575 B1 sind ZEN abbauende Eigenschaften von Bakterien der Gattung *Rhodococcus* und *Nocardia,* insbesondere *R. globerulus, R. erythropolis* und *N. globerula* bekannt.

Vekiru et al. (Appl. and Environ. Microb., 2010, 76, 7, 2353-2359) beschreibt die Biotransformation von ZEN in den weniger östrogenen Metaboliten ZOM-1 durch den Mikroorganismen *Trichosporon mycotoxinivorans.*

Die im Nachfolgenden verwendeten Ausdrücke sind der Fachsprache entnommen und werden jeweils, außer es ist etwas anderes angeführt, in den herkömmlichen Bedeutungen verwendet. So bezieht sich der Ausdruck "Polynukleotid" auf jegliche Arten von genetischem Material aller Längen und Sequenzen, wie z.B. Einzelstrang und Doppelstrang DNS- und RNS- Moleküle, inklusive regulatorischer Elemente, Strukturelemente, Gruppen von Genen, Plasmiden, gesamte Genome und Fragmente davon. Die Bezeichnung "Polypeptid" umfasst Proteine, wie beispielsweise Enzyme, Antikörper sowie Polypeptide mit bis zu 500 Aminosäuren, wie beispielsweise Peptidinhibitoren, Domänen von Proteinen aber auch kurze Polypeptide mit geringen Sequenzlängen, z.B. weniger als 10 Aminosäuren, wie Rezeptoren, Liganden, Peptidhormone, Tags und dgl. Die Bezeichnung "Position" in einem Polynukleotid oder Polypeptid bezieht sich auf eine einzelne, spezifische Base oder Aminosäure in der Sequenz des Polynukleotids oder des Polypeptids.

Die vorliegende Erfindung zielt nun darauf ab, ein Polypeptid zur Verfügung zu stellen, mit welchem es gelingt, Zearalenon schnell und zuverlässig in ein Zearalenon-Derivat umzuwandeln, dessen Toxizität und östrogene Wirkung so weit herabgesetzt ist, dass es für die jeweiligen Endnutzer gefahrlos in Futtermitteln oder Nahrungsmittprodukten verbleiben kann.

Zur Lösung dieser Aufgabe ist die vorliegende Erfindung im Wesentlichen dadurch gekennzeichnet, dass das Polypeptid eine die Ketogruppe an Position 7 von Zearalenon in eine Estergruppe umwandelnde Monooxygenase ist, und dass das Polypeptid eine Aminosäuresequenz, gewählt aus der Gruppe von Sequenz-ID Nrn. 1, 2 und 3 oder eine funktionelle Variante davon ist, wobei zwischen der funktionellen Variante und wenigstens einer der Aminosäuresequenzen wenigstens 60 %, bevorzugt wenigstens 70 %, noch bevorzugter wenigstens 80 % und am bevorzugtesten wenigstens 90 % Sequenzidentität besteht. Monooxygenasen sind zur Gruppe I der EC-Klassifikation gehörige Enzyme, die den Einbau eines Atoms Sauerstoff aus $O_2$ in das entsprechende Substrat, im vorliegenden Fall in Zearalenon katalysieren. Genauer wird im Falle von Zearalenon ein Sauerstoffatom an Position 7 der Ringstruktur des Zearalenons eingebaut, wodurch ein weniger stark toxisches Zearalenon-Derivat (iZOM) erhalten wird. Überraschenderweise hat sich herausgestellt, dass diese Detoxifizierung in nur einem einzigen enzymatischen Reaktionsschritt abläuft und dass das Reaktionsprodukt iZOM trotz der weiterhin vorhandenen geschlossenen Ringstruktur und der Kohlenstoff-Sauerstoff Doppelbindung an Position 7 eine um einen Faktor von ca. 100 geringere Toxizität besitzt und nahezu keine östrogene Wirkung mehr zeigt.

Eine besonders vollständige Umwandlung von Zearalenon in das weniger toxische Derivat iZOM gelingt, wenn das Polypeptid eine Aminosäuresequenz, gewählt aus der Gruppe von Sequenz ID-Nrn. 1, 2 und 3 oder eine funktionelle Variante davon ist, wobei zwischen der funktionellen Variante und wenigstens einer der Aminosäuresequenzen wenigstens 60 %, bevorzugt wenigstens 70 %, noch bevorzugter wenigstens 80 % und am bevorzugtesten wenigstens 90 % Sequenzidentität besteht. Durch das Vorhandensein von wenigstens einer derartigen konservierten Aminosäuresequenz, wie den Sequenzen der ID-Nrn. 1, 2 und 3 oder einer funktionellen Variante davon, gelingt es ein Polypeptid zur Verfügung zu stellen, das neben der schnellen und vollständigen Umwandlung von ZEN auch einen besonders hohen Aktivitätswert im Vergleich zu bis dato bekannten ZEN transformierenden Polypeptiden aufweist.

Bis dato wurde angenommen, dass für eine effiziente Detoxifizierung von ZEN eine Spaltung des Lactonringsystems erforderlich ist. Überraschenderweise hat sich jedoch herausgestellt, dass eine wirkungsvolle Detoxifizierung auch durch die Umwandlung der Ketogruppe an Position 7 von Zearalenon in eine Estergruppe erreichbar ist, bei welcher Umlagerung eine Spaltung des Ringsystems nicht erfolgt.

Gemäß einer Weiterbildung der Erfindung ist die Monooxygenase so gewählt, dass sie eine Baeyer-Villiger-Monooxygenase ist. Die Verwendung einer Baeyer-Villiger-Monooxygenase stellt sicher, dass die Reaktion ausschließlich an der gewünschten Position 7 stattfinden kann, da nur diese für eine sogenannte Baeyer-Villiger Oxidation, d.h. einen Umwandlung eines Ketons in einen Ester durch Einbau eines Sauerstoffatoms zugängig ist.

Hierbei ist das Polypeptid so gewählt, dass das Polypeptid, insbesondere die Monooxygenase, in einer einstufigen enzymatischen Oxidationsreaktion Zearalenon in iZOM umwandelt und dadurch in 24 Stunden und 30°C wenigstens 70 % des Zearalenons durch Umwandeln der Ketogruppe an Position 7 des Zearalenons in eine Estergruppe detoxifiziert, wobei das Polypeptid mittels einem transformierten Hefestamm YZGA515 der mit einem pCS57 Vektor der zusätzlich ein Polynukleotid zur Expression des Polypeptids enthält, transformiert ist, gebildet ist, und der transformierte Hefestamm in einer Zelldicht von OD600 von 4 eingesetzt ist und als Substrat Zearalenon in einer Konzentration von 2 mg/l eingesetzt ist, und als Reaktionsmedium SC-LEU Medium eingesetzt ist. Dies gelingt insbesondere deshalb, weil das Reaktionsprodukt iZOM überraschenderweise eine um einen Faktor von ca. 100 geringere Toxizität aufweist.

Aufgrund des konkreten Aufbaus des Zearalenon-Moleküls und insbesondere aufgrund der an Position 7 des Zearalenon-Moleküls vorhandenen Ketogruppe gelingt, wie dies einer Weiterbildung der Erfindung entspricht, der gezielte Einbau des gewünschten Sauerstoffatoms an Position 7 des Zearalenons mittels einer Untergruppe der Baeyer-Villiger-Monooxygenase, nämlich einer Cyclohexanon-Monooxygenase. Die durch diesen Einbau entstehende Estergruppe bewirkt überraschenderweise, dass der gebildete cyclische Metabolit (iZOM) eine deutlich geringere Toxizität als Zeralenon besitzt.

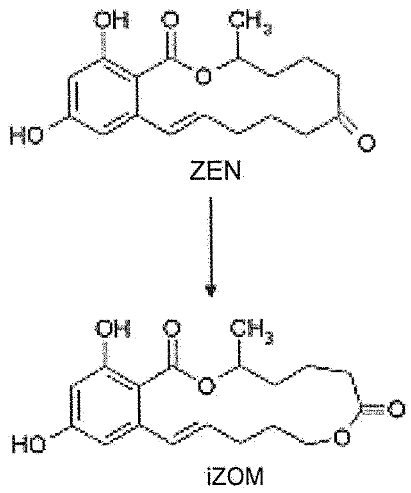


Unter dem Begriff "Sequenzidentität" wird eine prozentuale Sequenzidentität verstanden. Für Aminosäuresequenzen und Nukleotidsequenzen kann die Sequenzidentität untereinander visuell bestimmt werden, bevorzugt aber mit einem Computerprogramm errechnet werden. Als Referenzsequenz werden die Aminosäuresequenzen mit den Sequenz ID-Nrn. 1, 2 und 3 sowie die Nukleotidsequenzen mit den Sequenz ID-Nrn. 3, 4 und 5 definiert. Der Sequenzvergleich wird auch innerhalb von Sequenzabschnitten durchgeführt, wobei als Abschnitt eine durchgängige Sequenz der Referenzsequenz verstanden wird. Die Länge der Sequenzabschnitte beträgt für Nukleotidsequenzen normalerweise 18 bis 600, bevorzugt 45 bis 200, bevorzugter 100 bis 150 Nukleotide. Die Länge der Sequenzabschnitte beträgt für Peptidsequenzen normalerweise 3 bis 200, bevorzugter 15 bis 65, am bevorzugtesten 30 bis 50 Aminosäuren. Es gibt eine Vielzahl von käuflichen oder kostenfrei verfügbaren bioinformatischen Programmen die zur Homologiebestimmung herangezogen werden können und kontinuierlich weiterentwickelt werden. Beispiele hierfür sind GCG Wisconsin Bestfit package (Devereux et al. 1984), BLAST (Altschul et al. 1990) oder BLAST 2 (Tatusova und Madden 1999). Aufgrund der unterschiedlichen Einstellungsmöglichkeiten dieser Algorithmen ist es möglich, dass diese bei gleichen Inputsequenzen zu unterschiedlichen Ergebnissen kommen, weshalb der Suchalgorithmus und die dazugehörigen Einstellung definiert werden müssen. Im vorliegenden Fall wurde die Sequenzidentität mit Hilfe des Programms NCBI BLAST (Basic Local Alignment Search Tool), insbesondere mit BLASTP für Polypeptide und BLASTN für Polynukleotide, in der Version vom 20. Oktober 2014, welche auf der Homepage des "National Center for Biotechnology Information" (NCBI; http://www.ncbi.nlm.nih.gov/) zur Verfügung gestellt sind, durchgeführt. Damit ist es möglich zwei oder mehrere Sequenzen miteinander nach dem Algorithmus von Altschul et al., 1997 (Nucleic Acids Res., 25:3389-3402) zu vergleichen. Hierbei

wurden die Programme in der Version vom 15. Mai 2013 verwendet. Als Programmeinstellungen wurden die Basiseinstellungen herangezogen, insbesondere aber für den Aminosäuresequenzvergleich: "max target sequence" = 100; "expected treshold" = 10; "word size" = 3; "matrix" = BLOSOM62; "gap costs" = "Existence: 11; Extention: 1"; "computational adjustment" = "Conditional compositional score matrix adjustment"; sowie für den Nukleotidsequenzvergleich Word Size: 11; Expect value: 10; Gap costs: Existence = 5 , Extension = 2; Filter = low complexity activated; Match/Mismatch Scores: 2,-3; Filter String: L; m.

Die Ausdrücke "funktionelle Polypeptidvariante" oder "funktionelle Variante" beziehen sich zum Einen auf "allelische Varianten" des Polypeptids und auf "funktionelle Fragmente" des Polypeptids, zum Anderen auf ein "modifiziertes Polypeptid", wobei die enzymatische Funktion im Wesentlichen unverändert im Vergleich zum Polypeptid mit der Sequenz ID-Nr. 1 ist. Die Bezeichnung "allelische Variante" bezieht sich auf ein Polypeptid, das durch in der Natur zufällig vorkommende Mutation(en) der Nukleotidsequenz entstanden ist und eine Veränderung der Aminosäuresequenz bewirkt, wobei die enzymatische Funktion davon nicht beeinflusst ist. Der Ausdruck "funktionelles Fragment" bezieht sich auf einen Teil bzw. eine Teilsequenz eines Polypeptids oder einen Teil bzw. eine Teilsequenz einer funktionellen Variante davon, wobei die enzymatische Funktion im Wesentlichen beibehalten wird. "Modifizierte Polypeptide" können C- oder N-terminale Fusionensproteine oder gezielt mutierte Polypeptide sein, wobei Mutationen durch Substitution, Insertion oder Deletion von wenigstens einer Aminosäure, insbesondere durch ortsspezifische Mutagenese bzw. zufällige Mutagenese, Rekombination und/oder irgendein anderes "protein-engineering" Verfahren erhalten werden können, wobei die enzymatische Funktion im Wesentlichen beibehalten wird. Die Ausdrücke Substitution, Insertion und Deletion sind in der in der Gentechnik üblichen und dem Fachmann geläufigen Bedeutung verwendet. Eine enzymatische Funktion ist dann im Wesentlichen beibehalten, wenn der enzymatische Reaktionsmechanismus unverändert bleibt, d.h. die Ketogruppe an Position 7 des Mykotoxins ZEN in eine korrespondierende Estergruppe, wie im oben angeführten Reaktionsmechanismus gezeigt, umgewandelt wird und die spezifische Restaktivität wenigstens 5 %, bevorzugt wenigstens 10 %, insbesondere wenigstens 50 %, bezogen auf das ursprüngliche Polypeptid, beträgt.

Bei den Polypeptiden mit den Aminosäuresequenzen mit den Sequenz ID-Nrn. 1 und 3 handelt es sich um funktionelle allelische Varianten, wobei die Sequenzen jeweils aus unterschiedlichen Mikroorganismen stammen. Dies ist klar aus der nahen Verwandtschaft zueinander, gemessen mittels der prozentualen Sequenzidentität, erkennbar, sowie der Tatsache, dass die Polypeptide durch denselben Mechanismus auf ZEN einwirken. Das Polypeptid mit der Sequenz ID-Nr. 1 ist vollständig in der Sequenz mit der SEQ ID Nr. 2 enthalten, jedoch besitzt das Polypeptid mit der Sequenz ID-Nr. 2 einen um 28 Aminosäuren verlängerten N-Terminus.

Unter dem Begriff "Detoxifizierung" oder "detoxifizieren" wird die Reduktion der östrogenen Aktivität von ZEN, verstanden. Die Messung der östrogenen Aktivität erfolgt hierbei bevorzugt nach dem in den Ausführungsbeispielen beschriebenen Verfahren. Andere Verfahren können auch angewandt werden, wobei der entscheidende Faktor immer die Reduktion der östrogenen Aktivität von ZEN durch dessen enzymatische Umwandlung in iZOM ist. Gemessen mit dem in den Ausführungsbeispielen beschriebenen Östrogenitätsassay besitzt iZOM eine östrogene Aktivität die etwa um den Faktor 100 geringer als jene von ZEN ist.

Weiterhin kann ein isoliertes Polynukleotid zur Verfügung gestellt werden, mit welchem es gelingt ein Polypeptid zur schnellen und zuverlässigen Detoxifizierung von ZEN herzustellen. Hierzu besitzt das isolierte Polynukleotid eine Nukleotidsequenz, die für ein Polypeptid, welches eine die Ketogruppe an Position 7 von Zearalenon in eine Estergruppe umwandelnde Monooxygenase ist, codiert, und/oder einen Grad an Sequenzidentität zu wenigstens einer Nukleotidsequenz gewählt aus der Gruppe von Sequenz- ID Nr. 4, 5 und 6 von mindestens 60 % aufweist. Hierbei kann die Nukleotidsequenz unter mittleren Stringenzbedingungen mit wenigstens einer aus der Gruppe der Sequenz ID-Nrn. 4 bis 6 gewählten Nukleotidsequenz und/oder mit einer Teilsequenz davon von wenigstens 200 Nukleotiden, insbesondere von wenigstens 100 Nukleotiden und/oder mit einem komplementären Strang der Nukleotidsequenz oder Teilsequenz davon hybridisieren. Durch die Expression eines solchen Polynukleotids kann sichergestellt werden, dass das resultierende Polypeptid ZEN in iZOM transformiert.

Zu exprimierende Nukleotidsequenzen, insbesondere deren Triplets (Codons) werden in der Regel je nach Wirtszelle verändert, so dass der Codon Bias je nach Wirtszelle optimiert wird. Dies resultiert darin, dass auch Polynukleotide mit einem Grad an Sequenzidentität von weit unter 80 %, aber auch unter 70 % oder unter 60 % ein und dasselbe Polypeptid codieren können. Der Sequenzvergleich zur Bestimmung des Grads an Sequenzidentität muss auch innerhalb von Sequenzabschnitten durchgeführt werden, wobei ein Abschnitt als durchgängige Sequenz der Referenzsequenz zu verstehen ist. Die Länge der Sequenzabschnitte beträgt für Nukleotidsequenzen normalerweise 15 bis 600.

Mit Hilfe der vorliegenden isolierten Nukleotidsequenzen oder Sequenzabschnitte gelingt es, Nukleinsäuresonden, die eine Länge von in der Regel wenigstens 15, 30, oder 40 Nukleotiden besitzen, zu generieren. Mit Hilfe solcher als Sonden bezeichnete Nukleotidsequenzen, die meist zusätzlich markiert werden, z.B. mittels $^{3}H$, $^{32}P$, $^{35}S$, Biotin oder Avidin, können unter Anwendung von Standardmethoden Nukleotidsequenzen, die für Polypeptide mit ZEN abbauender Wirkung codieren, identifiziert werden. Als Ausgangsmaterial für die Identifikation solcher Sequenzen können zum Beispiel DNA, RNA oder cDNA von einzelnen Mikroorganismen, genomische DNA-Bibliotheken oder cDNA-Bibliotheken herangezogen werden.

Für Nukleotidsequenzen mit einer Länge von wenigstens 100 Nukleotiden werden mittlere Stringenzbedingungen definiert als Vorhybridisierung und Hybridisierung bei 42°C in 5-fachen NaCl versehenen Na-EDTA Puffer (SSPE, 0,9 M NaCl, 60 mM $NaH_2PO_4$, 6 mM EDTA) enthaltend 0,3 % Natriumdodecylsulfat (SDS), 200 μg/ml gescherte und denaturierte Lachsspermien-DNA und 35 % Formamid, gefolgt von Standard Southern Blot Bedingungen, wobei das Trägermaterial am Ende dreimal für 15 Minuten mit 2-fach Natriumchlorid-Zitratpuffer (SSC, 300 mM NaCl und 30 mM Trinatriumzitrat, 0,2 % SDS) bei 55°C gewaschen wird.

Für Nukleotidsequenzen mit einer Länge von 15 Nukleotiden bis 100 Nukleotiden werden mittlere Stringenzbedingungen definiert als vorhybridisieren und hybridisieren im Puffer bestehend aus 0,9 M NaCl, 0,09 M Tris-HCl pH = 7,6, 6 mM EDTA, 0,5% NP-40, 1-fach Denhardts Lösung, 1 mM Natriumpyrophosphat, 1 mM Natriumdihydrogenphosphat, 0,1 mM ATP und 0,2 mg/ml Hefe RNA, wobei bei einer Temperatur, die 5°C bis 10°C unter der errechneten Schmelztemperatur (Tm) liegt, vorhybridisiert und hybridisiert wird, wobei Tm durch Berechnung nach Bolton und McCarthy (1962, Proceedings of the National Academy of Sciences USA 48:1390) bestimmt wird. Im Anschluss wird der Versuch unter Standard Southern Blot Bedingungen weitergeführt (J. Sambrook, E.F. Fritsch und T. Maniatis, 1989, Molecular Cloning, A Laboratory Manual, 2nd edition, Cold Spring Harbor, New York). Das Trägermaterial wird am Ende einmal für 15 Minuten mit 6-fach SCC Puffer enthaltend 0,1% SDS und zweimal für 15 Minuten mit 6-fach SSC Puffer bei jeweils 5°C bis 10°C unter der errechneten Tm gewaschen.

Ein weiteres Ziel der Erfindung ist es eine transgene Wirtszelle zur Herstellung einer Zearalenon detoxifizierenden Monooxygenase zur Verfügung zu stellen.

Zur Lösung dieser Aufgabe ist die Erfindung im Wesentlichen, dadurch gekennzeichnet, dass die Wirtszelle ein Polynukleotid exprimiert, dass das Polynukleotid eine Nukleotidsequenz besitzt, die für wenigstens ein Polypeptid nach einem der Ansprüche 1 bis 3 codiert und einen Grad an Sequenzidentität zu wenigsten einer Nukleotidsequenz gewählt aus der Gruppe von Sequenz-ID Nr. 4, 5 und 6 von mindestens 60 % aufweist, wobei das Polynukleotid chromosomal integriert oder extrachromosomal vorliegt und die Wirtszelle eine Pflanzenzelle ist, und dass die Wirtszelle optional zusätzlich ein einen für die Oxygenase benötigten Cofaktor recycelndes, insbesondere NADP+ oder NAD+ in NADPH oder NADH umwandelndes Enzym überexprimiert.

$NADP^+$ oder $NAD^+$ in NADPH oder NADH umwandelnde Enzyme und dafür benötigte Cofaktoren können dem Stand der Technik, beispielsweise in Torres Pazmino et al. 2008 (Angew. Chem. 47(12); 2275-8), entnommen werden. Vorzugsweise sind die $NADP^+$ bzw. $NAD^+$ in NADPH bzw. NADH umwandelnden Enzyme so gewählt, dass diese in der Nahrungsmittel- und/oder Futtermittelindustrie problemlos eingesetzt werden können, insbesondere dass diese kompatibel mit den entsprechenden Nahrungsmittel- und/oder Futtermittelrechtlichen Vorschriften sind. Beispielsweise kann als $NADP^+$ in NADPH umwandelndes Enzym eine NADPH-abhängige Xylose Reduktase, insbesondere aus Pichia stipitis verwendet werden, die als Substrat das als Futtermittelzusatzstoff zugelassene Xylit verwendet. Alternativ dazu kann auch eine NAD(P)H umwandelnde Mannit Dehydrogenase aus Lactobacillus sp. eingesetzt werden, wobei als Substrat das als Futtermittelzusatzstoff zugelassene Mannit verwendet werden kann.

Vorzugsweise ist es möglich das Polypeptid zur Detoxifizierung von ZEN und ein zum Recyceln des Cofaktors befähigtes Enzym als Fusionsprotein herzustellen bzw. zu exprimieren. Dadurch können die beiden enzymatischen Aktivitäten in einem gemeinsamen Prozess, insbesondere einem biotechnologischen Up- und Downstreamprozess, hergestellt werden. Des Weiteren ist bei der Anwendung eines solchen Fusionsproteins sichergestellt, dass der zwischen den beiden Oxidationsstufen zirkulierende Cofaktor in räumlicher Nähe zum Polypeptid zur Detoxifizierung von ZEN verbleibt.

Mit einer derartigen transgenen Wirtszelle kann im Falle, dass die Wirtszelle eine Pflanzenzelle ist, Saatgut hergestellt werden, welches diese transgene Pflanzenzelle enthält.

Das Polypeptid kann in einer Zusammensetzung in verkapselter oder gecoateter Form vorliegen, wobei für das Verkapseln oder Coaten Standardmethoden, wie z.B. in der WO 92/12645 beschrieben, herangezogen werden können. Durch das Verkapseln bzw. Coaten gelingt es, das Polypeptid ohne Veränderung, insbesondere ohne Abbau und Schädigung an seinen Einsatzort zu transportieren, so dass erst nach Auflösung der Schutzhülle, beispielsweise im Verdauungstrakt von Tieren, das Polypeptid zu wirken beginnt, wodurch eine noch gezieltere, raschere und vollständigere Transformation von ZEN auch im sauren, proteasereichen und anaeroben Milieu erzielt werden kann. Des Weiteren gelingt es durch die Verkapselung oder das Coaten auch die Temperaturstabilität des Polypeptids im Zusatzstoff zu erhöhen.

Das verkapselte oder gecoatete Polypeptid kann mit in der Futtermittelindustrie üblichen Hilfsmitteln zu Prämixen weiterverarbeitet werden.

Weiterhin zielt die Erfindung auf die Verwendung eines isolierten Polynukleotids das für wenigstens ein Polypeptid gemäß der Erfindung codiert und einen Grad an Sequenzidentität zu wenigsten einer Nukleotidsequenz gewählt aus der Gruppe von Sequenz-ID Nr. 4, 5 und 6 von mindestens 60 % aufweist zur Herstellung einer Zearalenon detoxifizierenden Monooxygenase, ebenso wie auf eine Verwendung eines isolierten Polynukleotids das für wenigstens ein Polypeptid gemäß der Erfindung codiert und einen Grad an Sequenzidentität zu wenigstens einer Nukleotidsequenz gewählt aus der Gruppe von Sequenz-ID Nrn. 4, 5 und 6 von mindestens 60 % aufweist in einem Verfahren zur Herstellung

einer Zearalenon detoxifizierenden Monooxygenase.

Die vorliegende Erfindung zielt des Weiteren auf eine Verwendung des Zusatzstoffes zur Detoxifizierung von Zearalenon in Futtermitteln, insbesondere für Schweine, Geflügel und Aquakultur; in Nahrungsmitteln; oder in Trockenschlempe ab. Durch die erfindungsgemäße Verwendung des Zusatzstoffes gelingt es das im Nahrungs- oder Futtermittel bzw. in Trockenschlempe enthaltene ZEN durch Umwandeln der Ketogruppe an Position 7 in eine Estergruppe zu iZOM zu oxidieren und somit zu detoxifizieren, wobei eine derartige Entgiftung bereits bei niedrigen Polypeptidkonzentrationen in kontaminiertem Futter- oder Nahrungsmittel gelingt.

Hierbei liegt die Menge an Polypeptid pro Tonne Futter- oder Nahrungsmittel bzw. in Trockenschlempe in einem Bereich von ca. 1 g bis ca. 500 g, das verkapselte bzw. gecoatete Polypeptid in einem Bereich von ca. 20 g bis ca. 3000 g, der Prämix in einem Bereich von ca. 200 g bis ca. 10 kg oder der Zusatzsoff in einem Bereich von ca. 5 mg bis ca. 10 kg vor. Dadurch wird ermöglicht, dass das in Futter- oder Nahrungsmittel vorhandene ZEN, das darin bis zu einer Konzentration von 10.000 μg/kg vorliegen kann, zum überwiegenden Teil in iZOM umgewandelt wird.

Wird der Zusatzstoff in Verflüssigungsprozessen von Stärke, in Saccharifizierungsprozessen, in Biogasprozessen oder in Fermentationsprozessen, wie zum Beispiel im Maisch- oder Gärprozess der Bioethanolherstellung, eingesetzt so kann sichergestellt werden, dass ZEN in den für die Prozesse eingesetzten oder daraus entstehenden Produkten, wie zum Beispiel Trockenschlempe oder Stärke, in iZOM umgewandelt wird, wodurch keine gesundheitsschädlichen Mengen an ZEN intakt bleiben.

Die vorliegende Erfindung zielt des Weiteren darauf ab, ein Verfahren zur Herstellung einer Zearalenon detoxifizierenden Monooxygenase zur Verfügung zu stellen, mit dem eine schnelle und zuverlässige Überführung von ZEN in ein nicht-toxisches Derivat, nämlich iZOM, ermöglicht wird.

Zur Lösung dieser Aufgabe ist das Verfahren so geführt, dass ein isoliertes Polynukleotid, welches eine Nukleotidsequenz besitzt, die für wenigstens ein Polypeptid nach einem der Ansprüche 1 bis 3 codiert und/oder einen Grad an Sequenzidentität zu wenigsten einer Nukleotidsequenz gewählt aus der Gruppe von Sequenz-ID Nrn. 4, 5 und 6 von mindestens 60 % aufweist in eine transgene Wirtszelle chromosomal integriert oder extrachromosomal vorgelegt wird, dass die Wirtszelle eine prokaryontische oder eukaryontische Zelle, insbesondere eine Hefe- oder Pflanzenzelle ist und dass in der Wirtszelle optional zusätzlich ein einen für die Oxygenase benötigten Cofaktor recycelndes, insbesondere NADP+ oder NAD+ in NADPH oder NADH umwandelndes Enzym überexprimiert wird. Weiterhin kann ein derartiges isoliertes Polynukleotid, welches für ein Zearalenon detoxifizierendes Polypeptid codiert, zur Herstellung einer Wirtszelle verwendet werden, derart, dass die Wirtszelle das isolierte Polynukleotid, welches eine Nukleotidsequenz besitzt, die für wenigstens ein Polypeptid nach einem der Ansprüche 1 bis 4 codiert und/oder einen Grad an Sequenzidentität zu wenigsten einer Nukleotidsequenz gewählt aus der Gruppe von Sequenz-ID Nrn. 4, 5 und 6 von mindestens 60 % aufweist, exprimiert, wobei das Polynukleotid chromosomal integriert oder extrachromosomal vorgelegt wird und die Wirtszelle eine prokaryontische oder eukaryontische Zelle, insbesondere eine Hefe- oder Pflanzenzelle, ist und dass die Wirtszelle optional zusätzlich ein einen für die Oxygenase benötigten Cofaktor recycelndes, insbesondere NADP+ oder NAD+ in NADPH oder NADH umwandelndes Enzym überexprimiert.

Indem das Zearalenon durch eine Zugabe eines Polypeptids, insbesondere einer Monooxygenase, in einer einstufigen enzymatischen Oxidationsreaktion in iZOM umgewandelt und dadurch detoxifiziert wird, wobei die Ketogruppe an Position 7 des Zearalenons in eine Estergruppe umgewandelt wird und dass gegebenenfalls ein bei der Reaktion oxidierter Cofaktor, insbesondere NADP+ oder NAD+ mittels einem weiteren Enzym in NADPH oder NADH reduziert, wird nicht nur ein schneller Einbau des Sauerstoffatoms, welches gemeinsam mit der Ketogruppe in Position 7 die Estergruppe ausbildet gewährleistet sondern vor allem sichergestellt, dass die Reaktion vollständig abläuft und Zearalenon vollständig in das um etwa einen Faktor 100 weniger toxische iZOM umgewandelt wird. $NADP^+$ oder $NAD^+$ können dabei durch die bereits beschriebenen enzymatischen Systeme in NADPH oder NADH umwandelnde bzw. recycelt werden.

Hierbei kann als Polypeptid zur enzymatischen Detoxifizierung von Zearalenon ein Polypeptid mit einer Aminosäuresequenz gewählt aus den Sequenz ID-Nrn. 1, 2 und 3, oder einer funktionellen Variante davon eingesetzt werden, wobei eine Sequenzidentität zwischen der funktionellen Variante und wenigstens einer der Aminosäuresequenzen wenigstens 60 % beträgt. Bei Einsatz eines Polypeptids mit einer Aminosäuresequenz gewählt aus den Sequenz ID-Nrn. 1, 2 und 3 wird eine besonders vollständige Detoxifizierung von Zearalenon beobachtet.

Das erfindungsgemäße Verfahren kann hierbei sowohl in einem Zusatzstoff per se als auch in einer transgenen Wirtszelle, einer transgenen Pflanze oder in Saatgut eingesetzt werden und führt unabhängig vom Einsatzort zu gleichbleibend guten Ergebnissen und insbesondere einer vollständigen Transformation von Zearalenon in iZOM.

Das Verfahren kann hierbei so geführt werden, dass das Polypeptid oder der Zusatzstoff mit einem mit Zearalenon kontaminierten Futter- oder Nahrungsmittel vermischt wird, dass die erhaltene Mischung mit Feuchtigkeit in Kontakt gebracht wird, und dass das Polypeptid oder der Zusatzstoff das im kontaminierten Futter- oder Nahrungsmittel enthaltene Zearalenon detoxifiziert. Bei feuchten Futter- oder Nahrungsmitteln, wie Maische oder Breien findet, wird die Umwandlung von Zearalenon in iZOM vor der oralen Aufnahme im Futter- oder Nahrungsmittel statt, wodurch sichergestellt werden kann, dass die schädigenden Wirkungen von Zearalenon auf Mensch und Tier weitestgehend eliminiert

werden. Als Feuchtigkeit wird hierbei die Anwesenheit von Wasser oder wasserhältigen Flüssigkeiten verstanden, wobei darunter auch z.B. Speichel oder andere im Verdauungstrakt vorhandene Flüssigkeiten fallen.

Das erfinderische Verfahren kann auch so geführt werden, dass das Futter- oder Nahrungsmittel vor der oralen Aufnahme pelletiert wird.

Gemäß einer Weiterbildung der Erfindung ist das Verfahren zur Herstellung einer transgenen Wirtszelle so geführt, dass ein isoliertes Polynukleotid, welches eine Nukleotidsequenz besitzt, die für wenigstens ein Polypeptid nach einem der Ansprüche 1 bis 4 codiert und/oder einen Grad eine Sequenzidentität zu wenigstens einer Nukleotidsequenz gewählt aus der Gruppe von Sequenz-ID Nrn. 4, 5 und 6 von mindestens 60 % aufweist, in die Wirtszelle chromosomal integriert oder extrachromosomal vorgelegt wird, dass in der Wirtszelle das Polynukleotid exprimiert wird, dass als Wirtszelle eine prokaryontische oder eukaryontische Zelle, insbesondere eine Hefe- oder Pflanzenzelle eingesetzt wird und in der Wirtszelle optional zusätzlich ein einen für die Oxygenase benötigten Cofaktor recycelndes, insbesondere NADP+ oder NAD+ in NADPH oder NADH umwandelndes Enzym überexprimiert wird.

Ebenso bezieht sich die Erfindung auf die Verwendung einer transgenen Wirtszelle zur Herstellung einer Zearalenon detoxifizierenden Monooxygenase, welche dadurch gekennzeichnet ist, dass ein isoliertes Polynukleotid welches eine Nukleotidsequenz besitzt, die für wenigstens ein Polypeptid nach einem der Ansprüche 1 bis 4 codiert und/oder einen Grad eine Sequenzidentität zu wenigstens einer Nukleotidsequenz gewählt aus der Gruppe von Sequenz-ID Nrn. 4, 5 und 6 von mindestens 60 % aufweist, in die Wirtszelle chromosomal integriert oder extrachromosomal vorgelegt wird, dass in der Wirtszelle das Polynukleotid exprimiert wird, dass als Wirtszelle eine prokaryontische oder eukaryontische Zelle verwerndet wird, insbesondere eine Hefe- oder Pflanzenzelle und dass in der Wirtszelle optional zusätzlich ein einen für die Oxygenase benötigten Cofaktor recycelndes, insbesondere NADP+ oder NAD+ in NADPH oder NADH umwandelndes Enzym überexprimiert wird.

Die Erfindung wird nachfolgend anhand von Ausführungsbeispielen sowie Zeichnungen näher erläutert. In diesen zeigt:

Fig. 1 die teilweise Umwandlung von ZEN in der Negativkontrolle durch *S. cerevisiae.*

Fig. 2 die Umwandlung von ZEN im Versuchsansatz durch *S. cerevisiae* welche das Polypeptid mit der Sequenz ID-Nr. 1 exprimiert.

Fig. 3 die chemische Struktur von iZOM.

Fig. 4 die östrogene Aktivität von ZEN (Fig. 4A) sowie von iZOM (Fig. 4B).

Wenn nicht genauer angegeben, wurden alle molekularbiologischen bzw. mikrobiologischen Arbeiten unter Verwendung von Standardmethoden durchgeführt (Methods in Enzymology, Volume 194, Pages 3-933 (1991); Guide to Yeast Genetics and Molecular Biology. Edited by Christine Guthrie, Gerald R. Fink. ISBN: 978-0-12-182095-4; J. Sambrook et al. 2012, Molecular Cloning, A Laboratory Manual 4th Edition, Cold Spring Harbor).

Beispiel 1: Bildung von iZOM aus ZEN in einer einstufigen Reaktion

Der Hefestamm YZGA515 (Saccharomyces cerevisiae, modifiziert durch Inaktivierung des ABC Transportergens *PDR5* (Poppenberger et al. 2003, J. Biol. Chem., 278 (48) 47905-14) zur verbesserten Aufnahme von Zearalenon (ZEN)) wurde mittels Standardmethoden mit dem Plasmid pCS57 transformiert. pCS57 ist ein vom Hefe-Expressionsplasmid pADH-FW (Mitterbauer et. al 2002, Appl. Environ. Microbiol.; 68 (3), 1336-1346) abgeleitetes Plasmid mit LEU2 als Selektionsmarker und ADH1 Promoter.

Im Versuchsansatz wurde der Hefestamm YZGA515 mit dem pCS57 Vektor transformiert, der zusätzlich das Polynukleotid mit der Sequenz-ID Nr. 4 als BamHI-Xhol Fragment nach dem starken konstitutiven ADH1-Promoter (Alkoholdehydrogenase 1) enthielt. Dadurch wurde die Bildung des Polypeptids mit der Sequenz-ID Nr. 1 ermöglicht.

Als Negativkontrolle wurde der Hefestamm YZGA515 mit dem leeren Vektor (pADH-FW ohne dem Polynukleotid mit der Sequenz-ID Nr. 4) transformiert.

Die transgenen Hefen (Versuchsansatz und Negativkontrolle) wurden in SC-LEU Medium (Sherman 1991, Methods Enzymol., 194, 3-21) angezüchtet, abzentrifugiert und in frischem SC-LEU Medium mit einer Konzentration von 2 mg/l ZEN resuspendiert, wobei in beiden Fällen eine Zelldichte von OD600 = 4 eingestellt wurde. Nach verschiedenen Inkubationszeiten wurden Proben gezogen, diese wurden mit 1 Volumen Methanol versetzt und dadurch die intrazellulären Prozesse gestoppt. Durch Zentrifugation wurde die Zellsuspension geklärt und die resultierenden Überstände zur Messung mittels HPLC-MS verwendet. Gemessen wurde wie zuvor beschrieben die Ausgangssubstanz Zearalenon (ZEN), sowie das von Trichosporon mycotoxinivorans gebildete Transformationsprodukt ZOM-1 (Vekuri et al. 2010, Appl. Environ. Microbiol. 76(7) 2353-9).

In der mit dem leeren Vektor transformierten Negativkontrolle wurde kein ZOM-1 und insbesondere kein iZOM gebildet. Die entsprechenden HPLC Analysedaten sind in Fig. 1 gezeigt, wobei die y-Achse Nanogramm pro Milliliter (ng/ml) und die x-Achse die Zeit in Stunden (h) anzeigt.

Dagegen wurde im Versuchsansatz Zearalenon deutlich schneller transformiert und es wurde ein neuer Metabolit, mit der erwarteten Masse des von Vekiru et al. (2010) postulierten Intermdiates, im Folgenden genannt iZOM identifiziert. Zudem war auch ZOM-1 detektierbar, was ein klarer Hinweis darauf ist, dass in geringem Ausmaß eine Hydrolyse von iZOM zu ZOM auftritt. Die entsprechenden HPLC Analysedaten sind in Fig. 2 gezeigt. Im Vergleich zur Negativkontrolle wurde eine viel geringere Menge an ZEN gemessen, wobei die y-Achse Nanogramm pro Milliliter (ng/ml) und die x-Achse die Zeit in Stunden (h) anzeigt.

In einem weiteren Versuchsansatz wurde zusätzlich der Metabolit iZOM quantitativ gemessen. In Tabelle 1 ist die molare Bilanz der Metabolisierung von ZEN gezeigt. Daraus ist deutlich zu sehen, dass bereits nach 6 Stunden der Großteil des eingesetzten Zearalenon (ZEN) in iZOM umgewandelt wurde. Der geringe Fehlbetrag in der Bilanz ist mit der Bildung von weiteren Metaboliten sowie der methodisch bedingten analytischen Unschärfen erklärbar. Diese Ergebnisse zeigen deutlich, dass das Polypeptid mit der Sequenz-ID Nr. 1 in der Lage ist ZEN in den Metaboliten iZOM umzuwandeln.

Tabelle 1: Molaren Bilanz der Metabolisierung von ZEN im Versuchsansatz

| Zeit [h] | ZEN [nM] | ZOM-1 [nM] | iZOM [nM] |
|---|---|---|---|
| 0 | 3364 | 0 | 0 |
| 6 | 172,1 | 0 | 2163,1 |
| 24 | 4,8 | 214,8 | 2068,2 |

Beispiel 2: Identifizierung der chemischen Struktur von iZOM

Die chemische Struktur des Metaboliten iZOM (Fig. 3) konnte mittels NMR Messungen des isolierten und aufgereinigten Metaboliten bestimmt werden. Für die Messung der NMR Spektren wurde eine ausreichende Mengen iZOM aus mehreren Litern des Kulturfiltrates einer Hefekultur (SC-LEU wie in Beispiel 1 beschrieben) hergestellt. Mittels Festphasenextraktion und anschließender präparativer HPLC wurde iZOM aufgereinigt.

Mit Hilfe der isolierten Referenzsubstanz für iZOM kann zum einen die Umwandlung von ZEN zu iZOM verfolgt werden (siehe Beispiel 1) und zum anderen die chemische Struktur mittels NMR aufgeklärt werden.

Die NMR Spektren zur Identifizierung von iZOM wurden in einer CD3OD Lösung mit einem Bruker Avance DRX-400 FT-NMR Spektrometer bei Raumtemperatur (20°C) mit einer 5 mm inversen Breitband z-Gradienten Sondenkopf erhalten. Die chemischen Verschiebungen wurden auf Basis der residualen Lösungsmittelresonanz (3,31 ppm für $^{1}$H NMR, 49,15 ppm für $^{13}$C NMR) etabliert. Alle Pulsprogramme wurden von der Bruker Softwarebibliothek entnommen. Die NMR Daten wurden mittels TopSpin 1.3 (Bruker BioSpin GmbH) ausgewertet. Die komplette Strukturermittlung und Signalzuordnung wurde basierend auf $^{1}$H, $^{13}$C-APT, $^{1}$H$^{1}$H Korrelationsspektroskopie (COSY), $^{1}$H$^{13}$C heteronuklearen Einfachquantumkorrelationsspektren (HSQC), und $^{1}$H$^{13}$C heteronuklearen Multibandkorrelationsspektren (HMBC) durchgeführt.

Die NMR-Spektroskopiedaten von iZOM zeigen zwei wesentliche Unterschiede verglichen mit ZEN. Diese bestätigten die Transformation der Ketogruppe in eine Estergruppe. Die Unterschiede sind im Wesentlichen die Verschiebung des Carbonyl Kohlenstoffatoms von 212 ppm in ZEN (charakteristisch für ein Keton) auf 175 ppm (charakteristisch für ein Carboxylsäurederivat) und die Verschiebung der benachbarten $CH_2$ Gruppe von 36 ppm und 2,90/2,30 ppm (Position 8 in ZEN) zu 65 ppm und 4,20/4,05 (Position 9 in iZOM) für $^{13}$C beziehungsweise $^{1}$H, verursacht durch das angrenzende Sauerstoffatom. Im Gegensatz zum offenen Ring von ZOM1 ist der makrozyklische Ring in iZOM weiterhin intakt, wie durch eine Langstreckenkorrelation zwischen C7 und H9 evident ist. Die NMR Daten sind in Tabelle 2 angegeben. Die chemische Struktur von iZOM ist in Fig. 3 gezeigt.

Tabelle 2: $^{1}$H und $^{13}$C NMR Daten zur Identifizierung von iZOM.

| Position | $^{1}$H | | $^{13}$C |
|---|---|---|---|
| | δ (ppm) | Multiplizität, J (Hz) | δ (ppm) |
| 1 | - | | 172.9 |
| 3 | 5.14 | m | 74.2 |
| 3-$CH_3$ | 1.36 | d, 6.2 | 20.2 |
| 4 | 1.65-1.80 | m | 36.5 |
| 5 | 1.90,1.76 | m | 23.1 |
| 6 | 2.30-2.45 | m | 35.8 |

(fortgesetzt)

| Position | $^1H$ | | $^{13}C$ |
|---|---|---|---|
| | δ (ppm) | Multiplizität, J (Hz) | δ (ppm) |
| 7 | - | | 175.4 |
| 9 | 4.18,4.05 | m | 64.6 |
| 10 | 1.75-1.90 | m | 28.9 |
| 11 | 2.30-2.50 | m | 30.8 |
| 12 | 5.93 | ddd, 15.4, 8.3, 6.1 | 132.4 |
| 13 | 7.00 | d, 15.4 | 133.2 |
| 14 | 6.40 | d, 2.2 | 109.6 |
| 15 | - | | 165.0 |
| 16 | 6.19 | d, 2.2 | 103.3 |
| 17 | - | | 165.6 |
| 18 | - | | 104.9 |
| 19 | - | | 143.9 |

Beispiel 3: Stark verminderte östrogenen Aktivität von iZOM im Vergleich zu ZEN

Der gereinigte Metabolit iZOM, wie in Beispiel 2 beschrieben, wurde dazu verwendet um dessen Toxizität, ausgedrückt in östrogener Aktivität, zu bestimmen und mit der von Zearalenon zu vergleichen.

Zur Messung der östrogenen Aktivität wurde ein speziell dafür hergestellter Reporter-Hefestamm verwendet, YZHB817 (Bachmann, H.: Phenotypic detection of zearalenone in Saccharomyces cerevisiae. Master thesis BOKU Vienna, 2003). Dieser Stamm ist abgeleitet von Yeast-two Hybrid Stamm PJ69-4a (MATa trp1-901 leu2-3,112 ura3-52 his3-200 gal4- (deleted) gal80(deleted) LYS2::GAL1-HIS3 GAL2-ADE2 met2::GAL7-lacZ) (James et al. 1996, Genetics; 144(4), 1425-1436). PJ69-4a wurde durch die Disruption der ABC Transporter PDR5 und SNQ2 weiterentwickelt. Stämme mit dieser Doppelmutation pdr5 und snq2 zeigen besonders hohe ZEN-Aufnahme (Mitterbauer et al. 2003, Appl. Environ. Microbiol.; 69(2), 805-811). Dieser Stamm wurde anschließend mit einem Expressionsvektor transformiert (pTK103), der die Produktion eines Fusionsproteins auslöst, das die hormonabhängige Aktivierungsdomäne des humanen Östrogenrezeptors alpha enthält und die DNA-Bindungsdomäne des Hefe Gal4 Proteins um den Stamm YZHB817 zu erhalten.

In Anwesenheit östrogener Substanzen induziert der Hefestamm YZHB817 die Expression des GAL7 lacZ Reportergens, dessen Produkt, das Enzym beta-Galactosidase, leicht durch Hydrolyse des chromogenen Substrates ONPG (ortho-Nitrophenyl-beta-Galactosid) bei 420 nm gemessen werden kann. (Current Protocols in Molecular Biology, Chapter 13, Yeast (Eds. Lundblad V, Struhl, K.)).

Es wurden 100 μl der östrogenen Testlösung (ZEN oder iZOM) in 50% Ethanol gelöst mit 1,9 ml der Hefekultur des Stammes YZGA817 in SC-TRP medium mit einer Zelldichte von $OD_{600}$=0,1 vermischt und bei 30° und 180 rpm für 18 Stunden inkubiert. Danach wurde die $OD_{600}$ der Kultur bestimmt und das Zellpellet (10 min, 13krpm) von 1 ml in 500 ml Z-Puffer resuspendiert und mit 25 μl Chloroform permeabilisiert. Die Enzymreaktion wurde durch Zugabe von 100 μl ONPG-Lösung (4 mg/ml) gestartet und nach Gelbfärbung durch Zugabe von 250 μl einer 1 M $Na_2CO_3$ Lösung gestoppt. Der Überstand wurde bei 420 nm gemessen und die "relative Units" (bezogen auf die eingesetzte Zellmenge) wurde berechnet als:

$$RU= (OD_{420} \text{ X } 1000) / (OD_{600} \text{ X Inkubationszeit in min})$$

Wie in Fig. 4 gezeigt, induziert ZEN im niedrigen ppb Bereich die Expression des GAL7-lacZ Reportergens, so sind etwa 45 ppb ZEN notwendig um 7,5 relative Units beta-Galactosidase zu exprimieren (Fig. 4 A). iZOM hingegen weist eine weit geringere Östrogenität auf. Im Vergleich wurden etwa 500 ppb benötigt um etwa denselben Effekt zu erzielen (Fig. 4 B). Daraus resultiert, dass iZOM im Vergleich zu ZEN annähernd um den Faktor 100 weniger östrogen wirkt.

Beispiel 4: Bestimmung der Sequenzidentität

Die Bestimmung der prozentualen Sequenzidentität der Polypeptide mit den Aminosäuresequenzen mit den Sequenz ID-Nrn. 1, 2 und 3 relativ zueinander wurde durch den Abgleich zweier Sequenzen zueinander mit Hilfe des

Programms BLAST (Basic Local Alignment Search Tool), insbesondere mit BLASTP, welches auf der Homepage des "National Center for Biotechnology Information" (NCBI; http://www.ncbi.nlm.nih.gov/) benutzt werden kann, durchgeführt. Damit ist es möglich zwei oder mehrere Sequenzen miteinander nach dem Algorithmus von Altschul et al., 1997 (Nucleic Acids Res. (1997) 25:3389-3402) zu vergleichen. Als Programmeinstellungen wurden die Basiseinstellungen herangezogen, insbesondere aber: "max target sequence" = 100; "expected treshold" = 10; "word size" = 3; "matrix" = BLOSOM62; "gap costs" = "Existence: 11; Extention: 1"; "computational adjustment" = "Conditional compositional score matrix adjustment". Die Sequenzidentität zwischen den Sequenzen mit der SEQ ID Nr. 1 und SEQ ID Nr. 3 beträgt 73 %.

## Patentansprüche

1. Polypeptid zur enzymatischen Detoxifizierung von Zearalenon, **dadurch gekennzeichnet, dass** das Polypeptid eine die Ketogruppe an Position 7 von Zearalenon in eine Estergruppe umwandelnde Monooxygenase ist und dass das Polypeptid eine Aminosäuresequenz, gewählt aus der Gruppe von Sequenz-ID Nrn. 1, 2 und 3 oder eine funktionelle Variante davon ist, wobei zwischen der funktionellen Variante und wenigstens einer der Aminosäuresequenzen wenigstens 60 %, bevorzugt wenigstens 70 %, noch bevorzugter wenigstens 80 % und am bevorzugtesten wenigstens 90 % Sequenzidentität besteht.

2. Polypeptid nach Anspruch 1, **dadurch gekennzeichnet, dass** die Monooxygenase eine Baeyer-Villiger-Monooxygenase ist.

3. Polypeptid nach Anspruch 1 oder 2, **dadurch gekennzeichnet, dass** die Monooxygenase eine Cyclohexanon-Monooxygenase ist.

4. Transgene Wirtszelle zur Herstellung einer Zearalenon detoxifizierenden Monooxygenase, **dadurch gekennzeichnet, dass** die Wirtszelle ein Polynukleotid exprimiert, dass das Polynukleotid eine Nukleotidsequenz besitzt, die für wenigstens ein Polypeptid nach einem der Ansprüche 1 bis 3 codiert und einen Grad an Sequenzidentität zu wenigsten einer Nukleotidsequenz gewählt aus der Gruppe von Sequenz-ID Nr. 4, 5 und 6 von mindestens 60 % aufweist, wobei das Polynukleotid chromosomal integriert oder extrachromosomal vorliegt und die Wirtszelle eine Pflanzenzelle ist, und dass die Wirtszelle optional zusätzlich ein einen für die Oxygenase benötigten Cofaktor recycelndes, insbesondere NADP+ oder NAD+ in NADPH oder NADH umwandelndes Enzym überexprimiert.

5. Saatgut enthaltend eine transgene Pflanzenzelle nach Anspruch 4.

6. Verwendung eines isolierten Polynukleotids das für wenigstens ein Polypeptid nach einem der Ansprüche 1 bis 3 codiert und einen Grad an Sequenzidentität zu wenigsten einer Nukleotidsequenz gewählt aus der Gruppe von Sequenz-ID Nr. 4, 5 und 6 von mindestens 60 % aufweist zur Herstellung einer Zearalenon detoxifizierenden Monooxygenase.

7. Verwendung eines isolierten Polynukleotids das für wenigstens ein Polypeptid nach einem der Ansprüche 1 bis 3 codiert und einen Grad an Sequenzidentität zu wenigstens einer Nukleotidsequenz gewählt aus der Gruppe von Sequenz-ID Nrn. 4, 5 und 6 von mindestens 60 % aufweist in einem Verfahren zur Herstellung einer Zearalenon detoxifizierenden Monooxygenase.

8. Verfahren zur Herstellung einer Zearalenon detoxifizierenden Monooxygenase, **dadurch gekennzeichnet, dass** ein isoliertes Polynukleotid, welches eine Nukleotidsequenz besitzt, die für wenigstens ein Polypeptid nach einem der Ansprüche 1 bis 3 codiert und/oder einen Grad an Sequenzidentität zu wenigsten einer Nukleotidsequenz gewählt aus der Gruppe von Sequenz-ID Nrn. 4, 5 und 6 von mindestens 60 % aufweist in eine transgene Wirtszelle chromosomal integriert oder extrachromosomal vorgelegt wird, dass die Wirtszelle eine prokaryontische oder eukaryontische Zelle, insbesondere eine Hefe- oder Pflanzenzelle ist und dass in der Wirtszelle optional zusätzlich ein einen für die Oxygenase benötigten Cofaktor recycelndes, insbesondere NADP+ oder NAD+ in NADPH oder NADH umwandelndes Enzym überexprimiert wird.

9. Verwendung eines isolierten Polynukleotids, welches für ein Zearalenon detoxifizierendes Polypeptid codiert, zur Herstellung einer transgenen Wirtszelle **dadurch gekennzeichnet, dass** die Wirtszelle das isolierte Polynukleotid, welches eine Nukleotidsequenz besitzt, die für wenigstens ein Polypeptid nach einem der Ansprüche 1 bis 3 codiert und/oder einen Grad an Sequenzidentität zu wenigsten einer Nukleotidsequenz gewählt aus der Gruppe von Sequenz-ID Nrn. 4, 5 und 6 von mindestens 60 % aufweist, exprimiert, wobei das Polynukleotid chromosomal integriert oder extrachromosomal vorgelegt wird und die Wirtszelle eine prokaryontische oder eukaryontische Zelle, insbesondere eine Hefe- oder Pflanzenzelle, ist und dass die Wirtszelle optional zusätzlich ein einen für die Oxygenase benötigten Cofaktor recycelndes, insbesondere NADP+ oder NAD+ in NADPH oder NADH umwandelndes Enzym überexprimiert.

10. Verfahren zur Herstellung einer transgenen Wirtszelle zur Herstellung einer Zearalenon detoxifizierenden Monooxygenase, **dadurch gekennzeichnet, dass** ein isoliertes Polynukleotid, welches eine Nukleotidsequenz besitzt, die für wenigstens ein Polypeptid nach einem der Ansprüche 1 bis 3 codiert und/oder einen Grad eine Sequenzidentität zu wenigstens einer Nukleotidsequenz gewählt aus der Gruppe von Sequenz-ID Nrn. 4, 5 und 6 von mindestens 60 % aufweist, in die Wirtszelle chromosomal integriert oder extrachromosomal vorgelegt wird, dass in der Wirtszelle das Polynukleotid exprimiert wird, dass als Wirtszelle eine prokaryontische oder eukaryontische Zelle, insbesondere eine Hefe- oder Pflanzenzelle eingesetzt wird und in der Wirtszelle optional zusätzlich ein einen für die Oxygenase benötigten Cofaktor recycelndes, insbesondere NADP+ oder NAD+ in NADPH oder NADH umwandelndes Enzym überexprimiert wird.

11. Verwendung einer transgenen Wirtszelle zur Herstellung einer Zearalenon detoxifizierenden Monooxygenase, **dadurch gekennzeichnet, dass** ein isoliertes Polynukleotid welches eine Nukleotidsequenz besitzt, die für wenigstens ein Polypeptid nach einem der Ansprüche 1 bis 3 codiert und/oder einen Grad eine Sequenzidentität zu wenigstens einer Nukleotidsequenz gewählt aus der Gruppe von Sequenz-ID Nrn. 4, 5 und 6 von mindestens 60 % aufweist, in die Wirtszelle chromosomal integriert oder extrachromosomal vorgelegt wird, dass in der Wirtszelle das Polynukleotid exprimiert wird, dass als Wirtszelle eine prokaryontische oder eukaryontische Zelle verwerndet wird, insbesondere eine Hefe- oder Pflanzenzelle und dass in der Wirtszelle optional zusätzlich ein einen für die Oxygenase benötigten Cofaktor recycelndes, insbesondere NADP+ oder NAD+ in NADPH oder NADH umwandelndes Enzym überexprimiert wird.

## Claims

1. A polypeptide for the enzymatic detoxification of zearalenone, **characterized in that** the polypeptide is a monooxygenase converting the keto group in position 7 of zearalenone into an ester group, and that the polypeptide comprises an amino acid sequence selected from the group consisting of SEQ ID Nos. 1, 2 and 3 or a functional variant thereof, the functional variant and at least one of the amino acid sequences having a sequence identity of at least 60%, preferably at least 70%, more preferably at least 80%, and most preferably at least 90%.

2. A polypeptide according to claim 1, **characterized in that** the monooxygenase is a Baeyer-Villiger monooxygenase.

3. A polypeptide according to claim 1 or 2, **characterized in that** the monooxygenase is a cyclohexanone monooxygenase.

4. A transgenic host cell for preparing a zearalenone-detoxifying monoxygenase, **characterized in that** the host cell expresses a polynucleotide, that the polynucleotide comprises a nucleotide sequence encoding at least one polypeptide according to any one of claims 1 to 3 and having a degree of sequence identity of at least 60% with at least one nucleotide sequence selected from the group consisting of SEQ ID Nos. 4, 5 and 6, said polynucleotide being chromosomally integrated or extrachromosomally present and the host cell being a plant cell, and that optionally the host cell additionally overexpresses an enzyme recycling a cofactor required for the oxygenase, in particular an enzyme converting NADP+ or NAD+ to NADPH or NADH, respectively.

5. Seed comprising a transgenic plant cell according to claim 4.

6. The use of an isolated polynucleotide encoding at least one polypeptide according to any one of claims 1 to 3 and having a degree of sequence identity of at least 60% with at least one nucleotide sequence selected from the group consisting of SEQ ID Nos. 4, 5 and 6 for preparing a zearalenone-detoxifying monooxygenase.

7. The use of an isolated polynucleotide encoding at least one polypeptide according to any one of claims 1 to 3 and having a degree of sequence identity of at least 60% with at least one nucleotide sequence selected from the group consisting of SEQ ID Nos. 4, 5 and 6 in a method for preparing a zearalenone-detoxifying monooxygenase.

8. A method for preparing a zearalenone-detoxifying monoxygenase, **characterized in that** an isolated polynucleotide comprising a nucleotide sequence encoding at least one polypeptide according to any one of claims 1 to 3 and/or having a degree of sequence identity of at least 60% with at least one nucleotide sequence selected from the group consisting of SEQ ID Nos. 4, 5 and 6 is chromosomally integrated into a transgenic host cell or extrachromosomally provided, that the host cell is a prokaryotic or eukaryotic cell, in particular a yeast cell or a plant cell, and that optionally an enzyme recycling a cofactor required for the oxygenase, in particular an enzyme converting NADP+ or NAD+ to NADPH or NADH, respectively, is additionally overexpressed in the host cell.

9. The use of an isolated polynucleotide encoding a zearalenone-detoxifying polypeptide for preparing a transgenic host cell, **characterized in that** the host cell expresses the isolated polynucleotide, which comprises a nucleotide

sequence encoding at least one polypeptide according to any one of claims 1 to 3 and/or having a degree of sequence identity of at least 60% with at least one nucleotide sequence selected from the group consisting of SEQ ID Nos. 4, 5 and 6, wherein the polynucleotide is chromosomally integrated or extrachromosomally provided and the host cell is a prokaryotic or eukaryotic cell, in particular a yeast cell or a plant cell, and that optionally the host cell additionally overexpresses an enzyme recycling a cofactor required for the oxygenase, in particular an enzyme converting NADP+ or NAD+ to NADPH or NADH, respectively.

**10.** A method for preparing a transgenic host cell for preparing a zearalenone-detoxifying monooxygenase, **characterized in that** an isolated polynucleotide comprising a nucleotide sequence encoding at least one polypeptide according to any one of claims 1 to 3 and/or having a degree of sequence identity of at least 60% with at least one nucleotide sequence selected from the group consisting of SEQ ID Nos. 4, 5 and 6 is chromosomally integrated into the host cell or extrachromosomally provided, that the polynucleotide is expressed in the host cell, that a prokaryotic or eukaryotic cell, in particular a yeast cell or a plant cell, is used as said host cell, and that optionally an enzyme recycling a cofactor required for the oxygenase, in particular an enzyme converting NADP+ or NAD+ to NADPH or NADH, respectively, is additionally overexpressed in the host cell.

**11.** The use of a transgenic host cell for preparing a zearalenone-detoxifying monooxygenase, **characterized in that** an isolated polynucleotide comprising a nucleotide sequence encoding at least one polypeptide according to any one of claims 1 to 3 and/or having a degree of sequence identity of at least 60% with at least one nucleotide sequence selected from the group consisting of SEQ ID Nos. 4, 5 and 6 is chromosomally integrated into the host cell or extrachromosomally provided, that the polynucleotide is expressed in the host cell, that a prokaryotic or eukaryotic cell, in particular a yeast cell or a plant cell, is used as said host cell, and that optionally an enzyme recycling a cofactor required for the oxygenase, in particular an enzyme converting NADP+ or NAD+ to NADPH or NADH, respectively, is additionally overexpressed in the host cell.

## Revendications

**1.** Polypeptide pour la détoxification enzymatique de zéaralénone, **caractérisé en ce que** le polypeptide est une monooxygénase transformant le groupe céto en position 7 de zéaralénone en un groupe ester, et que le polypeptide est une séquence d'acides aminés, choisie parmi le groupe de séquence ID n° 1, 2 et 3, ou une variante fonctionnelle de celle-ci, dans lequel au moins 60 %, de manière préférée au moins 70 %, de manière davantage préférée au moins 80 % et de manière préférée entre toutes au moins 90 % d'identité de séquence existe entre la variante fonctionnelle et au moins une des séquences d'acides aminés.

**2.** Polypeptide selon la revendication 1, **caractérisé en ce que** la monooxygénase est une Baeyer-Villiger monooxygénase.

**3.** Polypeptide selon la revendication 1 ou 2, **caractérisé en ce que** la monooxygénase est une monooxygénase de cyclohexanone.

**4.** Cellule hôte transgénique pour fabriquer une monooxygénase détoxifiant du zéaralénone, **caractérisée en ce que** la cellule hôte exprime un polynucléotide, que le polynucléotide possède une séquence nucléotidique, qui code au moins un polypeptide selon l'une des revendications 1 à 3 et présente un degré d'identité de séquence par rapport à au moins une séquence nucléotidique, choisie parmi le groupe de séquence ID n° 4, 5 et 6, d'au moins 60 %, dans laquelle le polynucléotide est intégré de manière chromosomique ou est présent de manière extrachromosomique et la cellule hôte est une cellule végétale, et que la cellule hôte surexprime en outre de façon optionnelle une enzyme recyclant un cofacteur nécessaire à l'oxygénase, en particulier transformant du NADP+ ou du NAD+ en NADPH ou en NADH.

**5.** Semence contenant une cellule végétale transgénique selon la revendication 4.

**6.** Utilisation d'un polynucléotide isolé, qui code au moins un polypeptide selon l'une des revendications 1 à 3 et présente un degré d'identité de séquence par rapport à au moins une séquence nucléotidique, choisie parmi le groupe de séquence ID n° 4, 5 et 6, d'au moins 60 % pour la fabrication d'une monooxygénase détoxifiant du zéaralénone.

**7.** Utilisation d'un polynucléotide isolé, qui code au moins un polypeptide selon l'une des revendications 1 à 3 et

présente un degré d'identité de séquence par rapport à au moins une séquence nucléotidique, choisie parmi le groupe de séquence ID n° 4, 5 et 6, d'au moins 60 %, dans un procédé pour la fabrication d'une monooxygénase détoxifiant du zéaralénone.

8. Procédé de fabrication d'une monooxygénase détoxifiant du zéaralénone, **caractérisé en ce qu'**un polynucléotide isolé, lequel possède une séquence nucléotidique, qui code au moins un polypeptide selon l'une des revendications 1 à 3 et/ou présente un degré d'identité de séquence par rapport à au moins une séquence nucléotidique, choisie parmi le groupe de séquence ID n° 4, 5 et 6, d'au moins 60 %, est intégré de manière chromosomique ou est présent de manière extrachromosomique dans une cellule hôte transgénique, que la cellule hôte est une cellule procaryote ou eucaryote, en particulier une cellule de levure ou une cellule végétale, et qu'en outre de façon optionnelle une enzyme recyclant un cofacteur nécessaire à l'oxygénase, en particulier transformant du NADP+ ou du NAD+ en NADPH ou en NADH est surexprimée dans la cellule hôte.

9. Utilisation d'un polynucléotide isolé, lequel code un polypeptide détoxifiant du zéaralénone, pour la fabrication d'une cellule hôte transgénique, **caractérisée en ce que** la cellule hôte exprime le polynucléotide isolé, lequel possède une séquence nucléotidique, qui code au moins un polypeptide selon l'une des revendications 1 à 3 et/ou présente un degré d'identité de séquence par rapport à au moins une séquence nucléotidique, choisie parmi le groupe de séquence ID n° 4, 5 et 6, d'au moins 60 %, dans laquelle le polynucléotide est intégré de manière chromosomique ou est présent de manière extrachromosomique et la cellule hôte est une cellule procaryote ou eucaryote, en particulier une cellule de levure ou une cellule végétale, et que la cellule hôte surexprime en outre de façon optionnelle une enzyme recyclant un cofacteur nécessaire à l'oxygénase, en particulier transformant du NADP+ ou du NAD+ en NADPH ou en NADH.

10. Procédé de fabrication d'une cellule hôte transgénique pour la fabrication d'une monooxygénase détoxifiant du zéaralénone, **caractérisé en ce qu'**un polynucléotide isolé, lequel possède une séquence nucléotidique, qui code au moins un polypeptide selon l'une des revendications 1 à 3 et/ou présente un degré d'identité de séquence par rapport à au moins une séquence nucléotidique, choisie parmi le groupe de séquence ID n° 4, 5 et 6, d'au moins 60 %, est intégré de manière chromosomique ou est présent de manière extrachromosomique dans la cellule hôte, que le polynucléotide est exprimé dans la cellule hôte, qu'est employée en tant que cellule hôte une cellule procaryote ou eucaryote, en particulier une cellule de levure ou une cellule végétale et en outre de façon optionnelle une enzyme recyclant un cofacteur nécessaire à l'oxygénase, en particulier transformant du NADP+ ou du NAD+ en NADPH ou en NADH est surexprimée dans la cellule hôte.

11. Utilisation d'une cellule hôte transgénique pour la fabrication d'une monooxygénase détoxifiant du zéaralénone, **caractérisée en ce qu'**un polynucléotide isolé, lequel possède une séquence nucléotidique, qui code au moins un polypeptide selon l'une des revendications 1 à 3 et/ou présente un degré d'identité de séquence par rapport à au moins une séquence nucléotidique, choisie parmi le groupe de séquence ID n° 4, 5 et 6, d'au moins 60 %, est intégré de manière chromosomique ou est présent de manière extrachromosomique dans la cellule hôte, que le polynucléotide est exprimé dans la cellule hôte, qu'une cellule procaryote ou eucaryote, en particulier une cellule de levure ou une cellule végétale, est utilisée en tant que cellule hôte, et qu'en outre de façon optionnelle une enzyme recyclant un cofacteur nécessaire à l'oxygénase, en particulier transformant du NADP+ ou du NAD+ en NADPH ou en NADH est surexprimée dans la cellule hôte.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0938575 B1
- WO 9212645 A
- AT 2015000138 W
- AT 5382015 A


### In der Beschreibung aufgeführte Nicht-Patentliteratur


- **METZLER.** *Mycotox. Res.,* 2011, vol. 27, 1-3
- **VEKIRU et al.** *Appl. and Environ. Microb.,* 2010, vol. 76 (7), 2353-2359
- **ALTSCHUL et al.** *Nucleic Acids Res.,* 1997, vol. 25, 3389-3402
- **BOLTON ; MCCARTHY.** *Proceedings of the National Academy of Sciences USA,* 1962, vol. 48, 1390
- **J. SAMBROOK ; E.F. FRITSCH ; T. MANIATIS.** Molecular Cloning, A Laboratory Manual. Cold Spring Harbor, 1989
- **TORRES PAZMINO et al.** *Angew. Chem.,* 2008, vol. 47 (12), 2275-8
- *Methods in Enzymology,* 1991, vol. 194, 3-933
- Guide to Yeast Genetics and Molecular Biology
- **J. SAMBROOK et al.** Molecular Cloning, A Laboratory Manual. Cold Spring Harbor, 2012
- **POPPENBERGER et al.** *J. Biol. Chem.,* 2003, vol. 278 (48), 4709-14
- **MITTERBAUER.** *Appl. Environ. Microbiol.,* 2002, vol. 68 (3), 1336-1346
- **SHERMAN.** *Methods Enzymol.,* 1991, vol. 194, 3-21
- **VEKURI et al.** *Appl. Environ. Microbiol.,* 2010, vol. 76 (7), 2353-9
- **BACHMANN, H.** Phenotypic detection of zearalenone in Saccharomyces cerevisiae. *Master thesis BOKU,* 2003
- **JAMES et al.** *Genetics,* 1996, vol. 144 (4), 1425-1436
- **MITTERBAUER et al.** *Appl. Environ. Microbiol.,* 2003, vol. 69 (2), 805-811
- Yeast. Current Protocols in Molecular Biology